# EUROPEAN PATENT SPECIFICATION

(11) **EP 3 897 609 B1**
(45) Date of publication and mention of the grant of the patent: **24.01.2024**
(21) Application number: 19831869.3
(22) Date of filing: 23.12.2019
(51) Int. Cl.: A61K 31/33, A61K 31/00, G01N 33/574

(54) **METHODS OF TREATMENT AND DIAGNOSIS OF TUMOURS**
VERFAHREN ZUR BEHANDLUNG UND DIAGNOSE VON TUMOREN
MÉTHODES DE TRAITEMENT ET DE DIAGNOSTIC DE TUMEURS

(30) Priority: 21.12.2018 GB 201820994
(43) Date of publication of application: 27.10.2021
(73) Proprietor: UEA Enterprises Limited, Norwich NR4 7TJ (GB)
(72) Inventor: GREEN, Darrell, Norwich Norfolk NR4 7TJ (GB); FRASER, William, Norwich Norfolk NR4 7TJ (GB)
(74) Representative: Haseltine Lake Kempner LLP
(86) International application number: PCT/GB2019/053694
(87) International publication number: WO 2020/128534

(56) References cited:
- WO-A1-2016/149667
- WO-A1-2019/189772
- Kang Yang ET AL: "Original Article BMPR2-pSMAD1/5 signaling pathway regulates RUNX2 expression and impacts the progression of dedifferentiated chondrosarcoma", , 15 June 2016 (2016-06-15), XP055675740, Retrieved from the Internet: URL:https://www.ncbi.nlm.nih.gov/pmc/artic les/PMC4937734/pdf/ajcr0006-1302.pdf [retrieved on 2020-03-11]
- JIANG DONG ET AL: "The overexpression of miR-30a affects cell proliferation of chondrosarcoma via targeting Runx2", TUMOR BIOLOGY, KARGER, BASEL, CH, vol. 37, no. 5, 23 November 2015 (2015-11-23), pages 5933-5940, XP036218922, ISSN: 1010-4283, DOI: 10.1007/S13277-015-4454-3 [retrieved on 2015-11-23]
- XIAOJUAN SUN ET AL: "HDAC4 Represses Vascular Endothelial Growth Factor Expression in Chondrosarcoma by Modulating RUNX2 Activity", JOURNAL OF BIOLOGICAL CHEMISTRY, vol. 284, no. 33, 14 August 2009 (2009-08-14), pages 21881-21890, XP055675745, US ISSN: 0021-9258, DOI: 10.1074/jbc.M109.019091
- GREEN ET AL: "Transcriptional plasticity of tRNA fragments and microRNAs can be remodulated in chondrosarcoma", JOURNAL OF BONE AND MINERAL RESEARCH, BLACKWELL SCIENCE, INC, US, vol. 32, no. 1, 30 November 2016 (2016-11-30), page S197, XP009518892, ISSN: 0884-0431

## Description

### TECHNICAL FIELD

The present invention relates generally to compositions for use in methods of treating a chondrosarcoma and/or metastatic cancer originating from a chondrosarcoma.

### BACKGROUND

Chondrosarcoma is a malignant group of cartilage matrix forming bone tumours with diverse morphological features and clinical behaviour. Chondrosarcoma is resistant to cytotoxic chemotherapy and radiotherapy, rendering curettage, resection and amputation as the principal treatments. For patients who present with metastatic disease or locally advanced tumours who are not candidates for surgery, the disease is fatal. It is therefore desirable to provide new or improved methods for treating and diagnosing cartilage matrix-forming bone tumours.

As discussed herein, the present inventors have surprisingly and advantageously identified numerous markers of cartilage matrix-forming bone tumours and consequently developed new methods for treating and diagnosing cartilage matrix-forming bone tumours in subjects. In particular, the present inventors have surprisingly and advantageously identified the involvement of RUNX2 and YBX1 in tumour development and progression in cartilage matrix-forming bone tumours.

WO 2016/149667, relates to inhibitors of RUNX2 activity and expression in order to treat cancer. Although other cancers are mentioned, this publication primarily relates to the treatment of breast cancer. Cartilage matrix-forming bone tumours are not mentioned since the present inventors were the first to identify the involvement of RUNX2 in the development and progression of cartilage matrix-forming bone tumours.

### BRIEF DESCRIPTION OF THE FIGURES

The present invention and disclosure are described herein with reference to the following non-limiting figures.

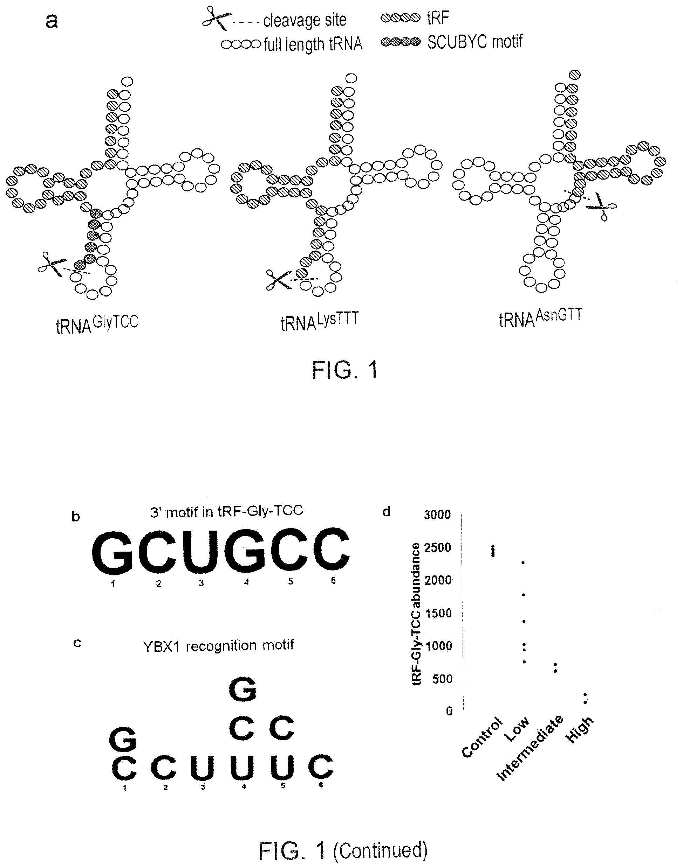

**Figure 1****.** Bioinformatics restricted to mature tRF sequences (**a**) full length tRNA secondary structures were generated using tRNA covariance model fold available on the genomic tRNA database. We show tRNA^{GlyTCC} with a 5' tRF cleavage between C and U at position 32, tRNA^{LysTTT} with a 5' cleavage between U and U at position 33 and tRNA^{AsnGTT} with a 3' cleavage between G and U at position 47 (**b**) sequence motif present in the 3' end of tRF-Gly-TCC first described in breast cancer as a YBX1 recognition sequence (**c**) the SCUBYC sequence motif that serves as a binding site for YBX1 (**d**) normalised count read matrix for tRF-Gly-TCC shows expression is steadily reduced in chondrosarcoma patients (each dot represents 1 patient).

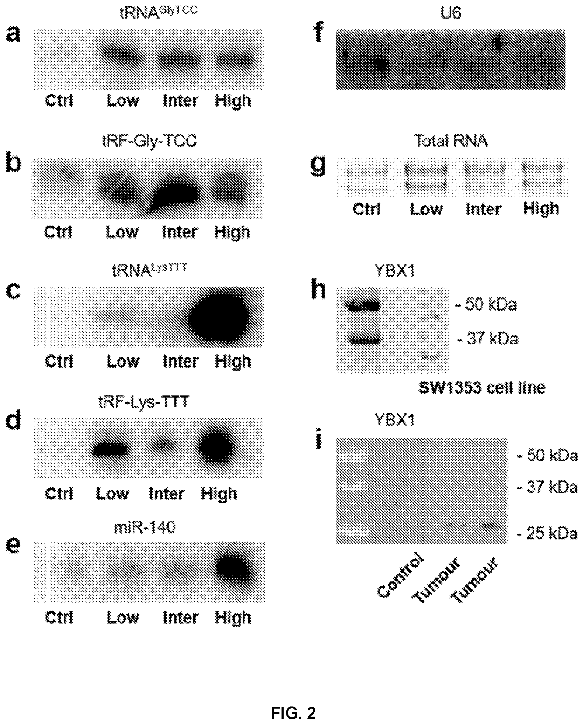

**Figure 2****.** Northern blot using antisense 3' end probes in control tissue (ctrl), low grade, intermediate grade and high grade chondrosarcoma tumours (**a**) tRNA^{GlyTCC} is upregulated in chondrosarcoma when compared to control (**b**) tRF-Gly-TCC is mostly upregulated in intermediate grade tumours and reduced to a lower level in high grade tumours (c) tRNA^{LysTTT} is markedly upregulated in high grade tumours (**d**) tRF-Lys-TTT has a high expression in low and high grade tumours (**e**) miR-140 is highly expressed in high grade tumours (**f** & **g**) U6 RNA and total RNA show equal loading to the membrane. Western blot using anti-YBX1 antibody in control tissue, chondrosarcoma cells and primary tumours shows (**h**) a 47 kDa variant and 30 kDa variant of YBX1 is expressed in the SW1353 chondrosarcoma cell line (**i**) a 27 kDa variant of YBX1 is upregulated in chondrosarcoma patient tumours.

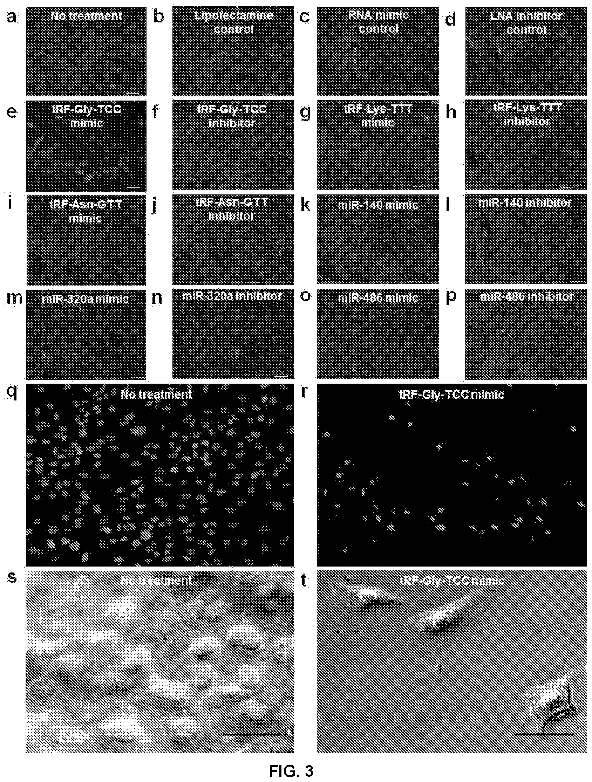

**Figure 3****.** Chondrosarcoma tumour spheres stained with 4', 6-diamidino-2-phenylindole (DAPI) to show nuclear DNA and Texas Red-X Phalloidin to show F-actin. Each tumour sphere was treated with a 50 nM concentration of tRF and miRNA mimic or inhibitor (**a**) untreated control (**b**) vehicle control (**c**) mimic control (**d**) inhibitor control (**e**) tRF-Gly-TCC mimic (**f**) tRF-Gly-TCC inhibitor (**g**) tRF-Lys-TTT mimic (**h**) tRF-Lys-TTT inhibitor (i) tRF-Asn-GTT mimic (j) tRF-Asn-GTT inhibitor (**k**) miR-140 mimic (**l**) miR-140 inhibitor (**m**) miR-320a mimic (**n**) miR-320a inhibitor (**o**) miR-486 mimic (**p**) miR-486 inhibitor (**q**) untreated tumour sphere nuclei are 488 ± 12.4 µm² (n=230) (**r**) tumour colony treated with a tRF-Gly-TCC mimic nuclei are 215 ± 16.8 µm² (n= 47) (**s**) overlay of nuclear DAPI staining on brightfield images of an untreated tumour sphere (**t**) overlay of nuclear DAPI staining on brightfield images of a tumour colony treated with a tRF-Gly-TCC mimic. Photomicrographs were taken at the most abundant cellular region of each tumour sphere. Scale bars are 100 µm (**a-p**) and 50 µm (**s** & **t**)**.**

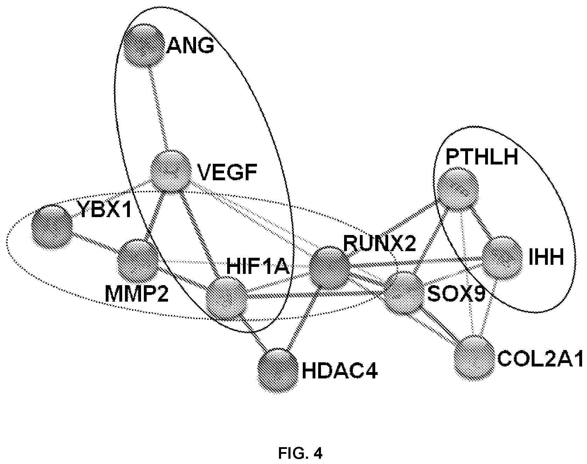

**Figure 4****.** Network of functional interactions between the genes involved in chondrogenesis and transformation, according to the literature, built using the STRING (Search Tool for the Retrieval of Interacting Genes/Proteins) database (v.10) at high confidence levels (scores between 0.4 and 0.9). The relative thickness of lines connecting proteins indicates the confidence score of their interaction. The solid black lines encircle the set of genes involved in hypoxic response. The dotted black line encircles the set of genes involved in metastasis. Protein nodes that are enlarged indicate the availability of 3D protein structure information. Embryonic miR-140 is a negative regulator of *RUNX2* and TRFs containing the SCUBYC motif interact with YBX1 to destabilise YBX1 bound oncogenic transcripts.

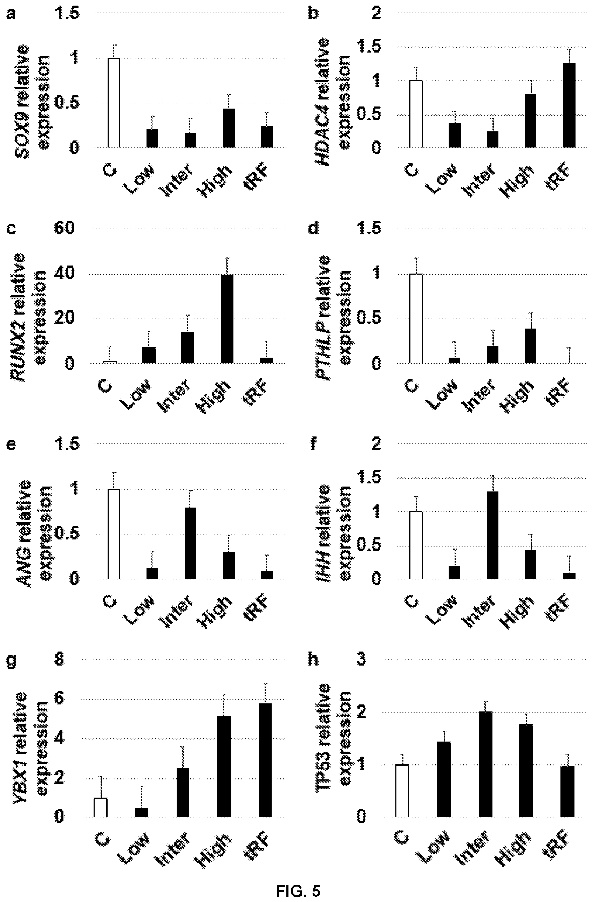

**Figure 5****.** Using the remaining RNA from previous experiments we performed qPCR based transcriptional profiling to investigate the mean relative expression of several genes displayed in Figure 5. We generated RNA pools of control tissue (C), low grade tumours, intermediate grade tumours (inter), high grade tumours and tumour spheres transfected with a tRF-Gly-TCC mimic (tRF). We show mean relative expression of (**a**) chondrocyte differentiation marker, *SOX9* (**b**) negative regulator of gene expression, *HDAC4* (**c**) embryonic regulator of chondrocyte and osteoblast cell proliferation and migration, *RUNX2* (**d**) marker of immature chondrocytes and propagator of the secondary ossification centre, *PTHLH* (**e**) regulator of angiogenesis and production of tRFs, *ANG* (**f**) regulator of chondrocyte differentiation, maturation and proliferation, *IHH* (**g**) RNA binding protein for stabilisation of oncogenic transcripts, *YBX1* (**h**) regulator of senescence and apoptosis, *TP53.* Data was normalised to *ACTB* expression and results are shown as mean ± SEM.

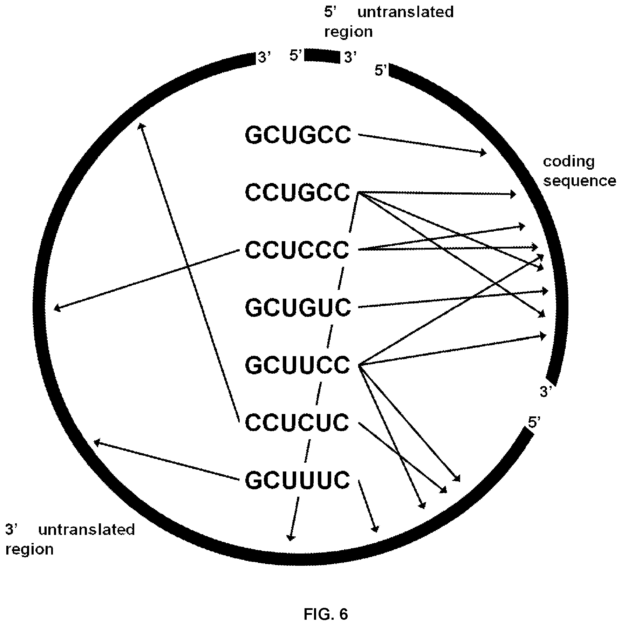

**Figure 6****.** Sequence analysis reveals several SCUBYC sequence motifs in the *RUNX2* transcript. YBX1 could bind to any of the presented motifs though previous reports would suggest YBX1 has preferential binding to sites in the 3' end.

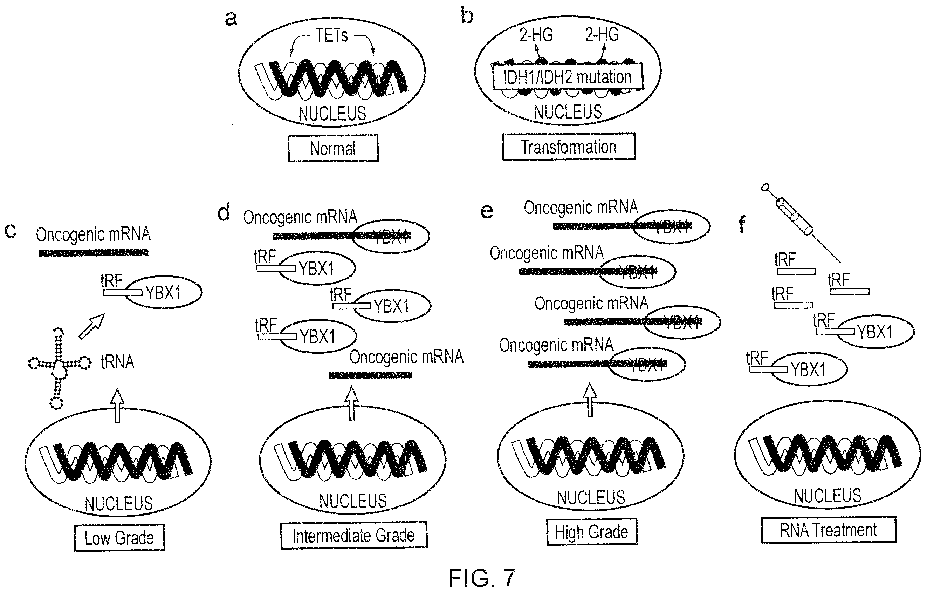

**Figure 7****.** Chondrosarcoma progression is proposed to be mediated by attenuation of specific tRFs that interact with and negatively regulate YBX1. (**a**) under normal cellular conditions, TETs prevent DNA hypermethylation (**b**) mutations in *IHD1* and/or *IDH2* result in a loss of catalytic activity leading to the abnormal production of 2-HG which accumulates and interferes with the activity of TETs. TETs can no longer prevent hypermethylation, which leads to chondrosarcoma development (**c**) malignant cells replicate and form a low grade tumour. TRNAs are cleaved into tRFs to prevent stabilisation of metastatic transcripts (**d**) intermediate grade tumours start to produce transcripts with metastatic propensity. TRFs containing the SCUBYC sequence motif interact with YBX1 to terminate the stability of oncogenic messenger RNAs (**e**) production of tRFs containing the SCUBYC motif is switched off, allowing YBX1 to stabilise metastatic transcripts and enable malignant cells to move out of the tumour and spread (**f**) exogenous delivery of tRF-Gly-TCC resolves tRF-YBX1 interaction and abates tumour progression.

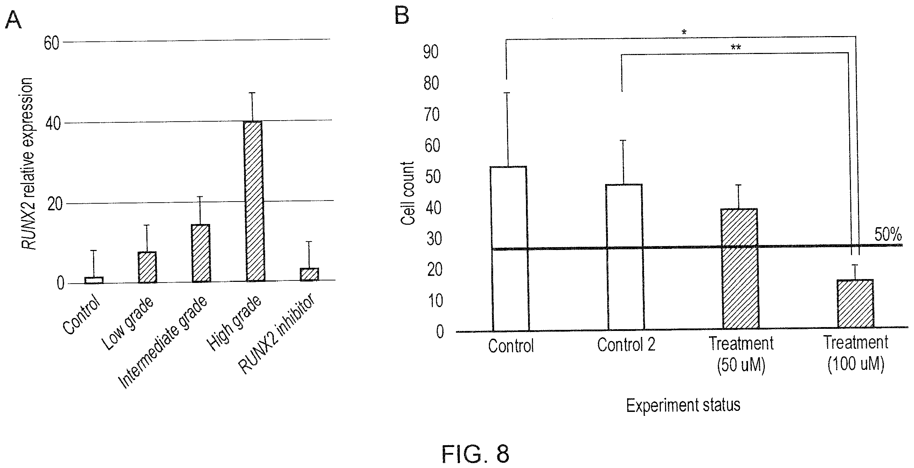

**Figure 8****.** (**a**) (based on Figure 5(c), for comparison) *RUNX2* expression in control tissue, low grade, intermediate grade and high grade chondrosarcoma tumours. Downregulation of *RUNX2* using an RNA inhibitor reduces *RUNX2* expression to control levels. (**b**) Inhibition of RUNX2 binding to DNA in bone 3D tumour spheres using the small molecule CAD522 leads to a significant reduction of cancer cell number compared to controls (two biological replicate controls). Statistical significance (**p* = <0.002 and ***p* = <0.005) was calculated using an unpaired t test with Prism 6 (GraphPad) software.

### BRIEF DESCRIPTION OF THE INVENTION

The invention is defined by the appended claims.

The present invention provides a composition for use in a method of treating a chondrosarcoma and/or a metastatic cancer originating from a chondrosarcoma, wherein the composition comprises an inhibitor of RUNX2 activity, wherein the inhibitor of RUNX2 activity is a compound according to formula (I) or a pharmaceutically acceptable salt, ester or prodrug thereof, 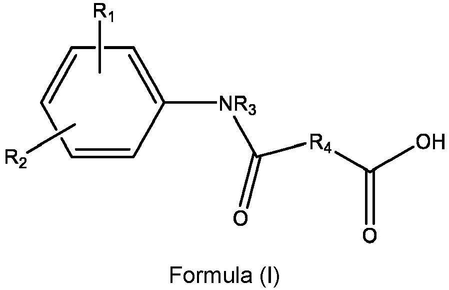
wherein R₁ and R₂ are each independently selected from hydrogen, a halogen, a haloalkyl, an alkyl, an alkylamide, a cycloalkylamide or an alkyamine;
R₃ is H or alkyl; and
R₄ is a bridged cycloalkenyl ring.In the present invention, the chondrosarcoma can overexpress RUNX2 in comparison to normal cartilage or other biological material.

In the present invention the compound of Formula (I) can be 3-(N-(3,4-dichlorophenyl)carbamoyl)-5-norbornene-2-carboxylic acid, also named 3-{[(3,4-dichlorophenyl)amino]carbonyl}bicyclo[2.2.1]hept-5-ene-2-carboxylic acid, known as CADD522 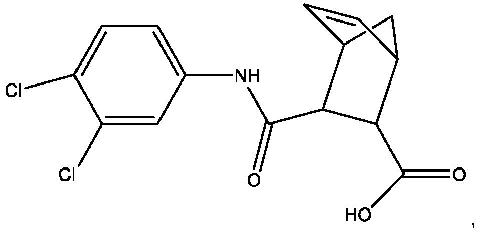 or a salt, ester or prodrug thereof.

This disclosure also relates to a composition for use in a method of treating a cartilage matrix-forming bone tumour or a metastatic cancer originating from a cartilage matrix-forming bone tumour, wherein the composition comprises one or more of an inhibitor of RUNX2 activity, an inhibitor of RUNX2 expression, an inhibitor of YBX1 activity and an inhibitor of YBX1 expression.

The composition of the present invention may comprise, consist essentially of or consist of an inhibitor of RUNX2 activity. The composition may, for example, be a pharmaceutical composition comprising, consisting essentially of or consisting of one or more of an inhibitor of RUNX2 activity and a pharmaceutically acceptable carrier and/or excipient and/or diluent.

As discussed herein, an inhibitor of RUNX2 activity and/or an inhibitor of YBX1 activity can inhibit binding of RUNX2 and/or YBX1 to DNA and/or mRNA. As discussed herein an inhibitor of RUNX2 activity and/or YBX1 activity can inhibit interaction of YBX1 protein with RUNX2 transcripts and/or RUNX2 protein.

In the present invention, the inhibitor of RUNX2 activity is a compound according to formula (I) or a pharmaceutically acceptable salt, ester or prodrug thereof, 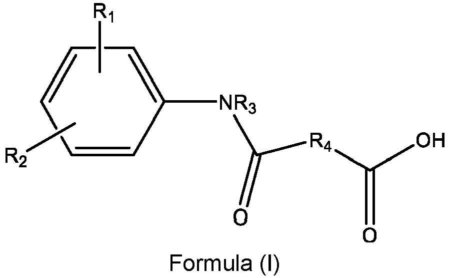
wherein R₁ and R₂ are each independently selected from hydrogen, a halogen, a haloalkyl, an alkyl (e.g. a C₁₋₃ alkyl straight chain alkyl or a C₅₋₇ cycloalkyl), an alkylamide, a cycloalkylamide or an alkyamine (e.g. a dialkylamine);
R₃ is H or alkyl; and
R₄ is a bridged cycloalkenyl ring (e.g. cyclohexene ring with an alkyl bridge such as a methylene bridge which may be optionally substituted, for example optionally substituted with a cycloalkyl ring such as a C₃₋₆cycloalkyl ring (for example, a cyclopropane ring)).

An inhibitor of YBX1 activity and/or an inhibitor of YBX1 expression may be a small RNA molecule comprising a SCUBYC motif. An inhibitor ofYBX1 activity and/or the inhibitor of YBX1 expression may be a tRNA derived fragment (tRF) or analogue thereof, for example tRF-Gly-TCC or an analogue thereof.

An inhibitor of RUNX2 expression and/or the inhibitor of YBX1 expression may also be a siRNA.

There is provided but not claimed an *in vitro* method of detecting the presence of a cartilage matrix-forming bone tumour in a subject or the risk of a subject developing a cartilage matrix-forming bone tumour, the method comprising (i) measuring the expression level of at least one of RUNX2 and YBX1 in a biological sample obtained from a subject, and (ii) comparing the expression level of RUNX2 and/or YBX1 in the biological sample obtained from the subject with the respective expression level of RUNX2 and/or YBX1 in normal cartilage or other (i.e. also normal) biological material, wherein a higher expression level of RUNX2 and/or YBX1 in the biological sample obtained from the subject compared to the respective expression level of RUNX2 and/or YBX1 in the normal cartilage or other biological material indicates the presence of or an increased risk of developing a cartilage matrix-forming bone tumour.

There is provided but not claimed an *in vitro* method of determining the prognosis of a subject having a cartilage matrix-forming bone tumour, the method comprising (i) measuring the expression level of at least one of RUNX2 and YBX1 in a biological sample obtained from the subject, and (ii) comparing the expression level of RUNX2 and/or YBX1 in the biological sample obtained from the subject with the respective expression level of RUNX2 and/or YBX1 in normal cartilage or other biological material, wherein the subject's prognosis decreases with increasing expression level of RUNX2 and/or YBX1 in the biological sample obtained from the subject.

The expression level of RUNX2 and/or YBX1 may each independently be determined by measuring the levels of protein and/or mRNA.

The method may comprise measuring the expression level of one or more tRF having a SCUBYC motif in the biological sample obtained from the subject such as tRF-Gly-TCC and/or tRF-Lys-TTT.

The method may comprise measuring the expression level of one or more of tRNA^{GlyTCC}, tRNA^{LysTTT}, miR-140, SOX9, PTHLP, ANG, HDAC4, and p53 in the biological sample obtained from the patient. The method comprises measuring the expression level of one or more of tRNA^{GlyTCC}, tRNA^{LysTTT}, miR-140, SOX9, PTHLP, ANG, and p53 in the biological sample obtained from the patient.

In the present invention, the cartilage matrix-forming bone tumour is a cartilage matrix-forming cancer. In the present invention, the cartilage matrix-forming bone cancer is chondrosarcoma. In certain embodiments of any aspect of the present invention, the metastatic cancer is in the lung.

In embodiments of any aspect of the present invention, the subject is an animal. In certain embodiments, the subject is a human subject.

The details, examples and preferences provided in relation to any particular one or more of the stated aspects or embodiments of the present invention apply equally to all other aspects or embodiments of the present invention.

### DETAILED DESCRIPTION

The present invention is based on the surprising finding that certain genes are differentially expressed during the development and progression of a cartilage matrix-forming tumour, in particular chondrosarcoma, in comparison to normal cartilage or other biological material. In particular, the present invention is based on the surprising finding that there is increased expression of RUNX2 and YBX1 in chondrosarcoma, in comparison to normal cartilage or other biological material, and that inhibition of RUNX2 binding to DNA in chondrosarcoma leads to significant reduction in chondrosarcoma cell numbers. These surprising findings have enabled the identification of new methods of treatment, methods of diagnosis and methods of prognosis of cartilage matrix-forming tumours and metastatic cancers originating from cartilage matrix-forming tumours, and compositions for use in these methods. These methods are particularly applicable to cartilage matrix-forming tumours and metastatic cancers originating from cartilage matrix-forming tumour that overexpress RUNX2 and/or YBX1 in comparison to normal cartilage or other biological material. Furthermore, the specific attribution of inhibition of protein (particularly RUNX2) binding to DNA as the underlying causation of the reduction in tumour and cancer cell number enables specific treatments to target that specific site in the matrix of pathways supporting tumour/cancer cell growth and maintenance, enabling reduced adverse or side effects and a better treatment outcome and treatment experience for patients. The term "normal cartilage or other biological material" refers particularly to non-tumour tissue, in particular non-cancerous cartilage tissue, or other biological material that is representative of the normal expression level of any relevant one or group of markers with which the present invention is concerned. The normal cartilage or other biological material may, for example, be obtained from a different subject to the subject of the method of treatment or diagnosis or prognosis. Alternatively, the normal cartilage or other biological material may be obtained from the same subject as the subject of the method of treatment or diagnosis or prognosis, but from a region that does not include tumour cells, particularly cancer cells. Thus, the normal cartilage or other biological material may, for example, be matched cartilage tissue from the subject, taken from before the onset or progression of symptoms of disease, matched cartilage tissue from the subject in which symptoms or disease are absent, or cartilage tissue from one or more healthy individual(s) other than the subject.

The term "expression level in normal cartilage or other biological material" includes, for example, the level in an individual sample or a statistically derived level from multiple samples from one or more individual(s) which is determined to be representative of the level in normal cartilage or other biological material in a population.

The term "expression level" herein refers generally to the level of occurrence (amount or concentration) of any expression product or combination of expression products, including product or products of one or both of transcription and translation of the respective gene or genes, with or without splicing, which expression product can be active or inactive or partially active.

The term "cartilage matrix-forming tumour" refers to any tumour that secretes a cartilage-like material. The cartilage matrix-forming tumour may be benign or cancerous. The cartilage matrix-forming tumour, for example the cartilage matrix-forming cancer, may, for example, arise from mesenchymal cells. Benign cartilage-matrix forming tumours may, for example, be selected from chondroma (e.g. enchondroma or ecchondroma), osteochondroma, chondroblastoma, chondromyxoid fibroma, and combinations thereof. Cartilage-matrix forming cancers may, for example, be selected from chondrosarcoma (including conventional, juxtacortical, mesenchymal, dedifferentiated and clear cell chondrosarcomas), fibroadenoma, and combinations thereof. The cartilage matrix-forming cancer may, for example, be chondrosarcoma. The cartilage matrix-forming tumour includes recurrent tumours.

The term "metastatic cancer originating from a cartilage matrix-forming bone tumour" refers to cancer in any part of the body that is formed from cartilage matrix-forming bone tumour cells that have spread from the primary tumour. The metastatic cancer originating from a cartilage matrix-forming bone tumour may, for example, be found in the lung(s) and/or brain of the subject. The metastatic cancer originating from the primary tumour may, for example, be found in other bones in the subject.

The subject may, for example, be an animal. The subject may, for example, be a human. Alternatively, the subject may be a mammal other than a human, such as non-human primates (e.g. apes, monkeys and lemurs), companion animals such as cats or dogs, working and sporting animals such as dogs, horses and ponies, farm animals such as pigs, sheep, goats, deer, oxen and cattle, and laboratory animals such as rodents (e.g. rabbits, rats, mice, hamsters, gerbils or guinea pigs).

### Methods of Treatment

There is therefore disclosed but not claimed herein a method of treating a cartilage matrix-forming bone tumour and/or a metastatic cancer originating from a cartilage matrix-forming bone tumour, the method comprising administering one or more of an inhibitor of RUNX2 activity, an inhibitor of RUNX2 expression, an inhibitor of YBX1 activity and an inhibitor of YBX1 expression, to a subject in need thereof. The present invention relates to compositions for use in a method of treating a chondrosarcoma and/or a metastatic cancer originating from a chondrosarcoma. The composition comprises an inhibitor of RUNX2 activity. The composition may also comprise one or more of an inhibitor of RUNX2 expression, an inhibitor of YBX1 activity and an inhibitor of YBX1 expression.

The term "treating" in relation to therapeutic method of treatment, also includes prophylaxis and the alleviation of symptoms of a disease and/or disorder in a subject. The expression "treating" and analogous terms used herein refers to all forms of healthcare intended to remove or avoid the disease and/or disorder or to relieve its symptoms, including preventive and curative care, as judged according to any of the tests available according to the prevailing medical practice. An intervention that aims with reasonable expectation to achieve a particular result but does not always do so is included within the expression "treating". An intervention that succeeds in slowing or halting progression of a disease and/or disorder is included within the expression "treating". The methods described herein may, for example, inhibit metastasis or further metastasis of a cartilage matrix-forming bone tumour.

The term "inhibitor of RUNX2/YBX1 activity" refers to any agent capable of preventing RUNX2/YBX1 from performing its role in the development and/or progression (including metastasis) of the cartilage matrix-forming bone tumour. The inhibitor of RUNX2/YBX1 activity may, for example, interact with the RUNX2/YBX1 DNA and/or RUNX2/YBX1 mRNA and/or RUNX2/YBX1 protein to prevent it from performing its role in the development and/or progression of the cartilage matrix-forming bone tumour. For example, the inhibitor of RUNX2/YBX1 may bind to the respective RUNX2/YBX1 protein in order to prevent the RUNX2/YBX1 protein from binding to DNA and/or mRNA. The inhibitor of RUNX2/YBX1 activity may, for example, inhibit the interaction of YBX1 and RUNX2, for example the interaction of YBX1 protein and RUNX2 transcripts and/or RUNX2 protein. In one particular embodiment of the present invention, the inhibitor of RUNX2 activity inhibits (preferably specifically and/or selectively inhibits) the binding of RUNX2 to DNA, for example in the tumour cells. In one particular embodiment of the present invention, the inhibitor of YBX1 activity inhibits (preferably specifically and/or selectively inhibits) the binding of YBX1 to DNA, for example in the tumour cells.

The term "inhibitor of RUNX2/YBX1 expression" refers to any agent capable of reducing expression of RUNX2/YBX1 in the cartilage matrix-forming bone tumour or metastatic cancer originating from a cartilage matrix-forming bone tumour. The inhibitor of RUNX2/YBX1 expression may, for example, reduce or prevent transcription of the DNA such that a reduced number of mRNA transcripts are formed. Alternatively or additionally, the inhibitor of RUNX2/YBX1 expression may reduce or prevent translation of the mRNA such that a reduced number of RUNX2/YBX1 proteins are formed. The inhibitor of RUNX2/YBX1 expression may, for example, be a small RNA molecule. The term "small RNA molecule" refers to an RNA molecule comprising less than about 35 nucleotides, for example comprising from about 14 to about 35 nucleotides, for example comprising from about 19 to about 35 nucleotides, for example comprising from about 19 to about 33 nucleotides. Small RNA molecules may be single or double stranded and include, for example, miRNAs, piRNAs, siRNAs, tRNAs, tRNA fragments (tRFs), molecules that mimic endogenous RNA molecules, and combinations of one or more thereof. The inhibitor of RUNX2/YBX1 expression may also be a siRNA molecule.

The inhibitor of RUNX2 activity and/or the inhibitor of RUNX2 expression is a compound according to formula (I) or a pharmaceutically acceptable salt, ester or prodrug thereof, 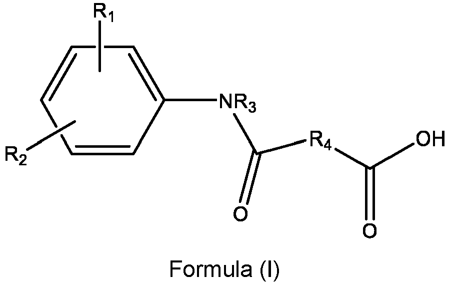
wherein R₁ and R₂ are each independently selected from hydrogen, a halogen (e.g. CI), an alkyl (e.g. a C₁₋₃ alkyl straight chain alkyl or a C₅₋₇ cycloalkyl), a haloalkyl, an alkylamide, a cycloalkylamide or an alkyamine (e.g. a dialkylamine);
R₃ is H or alkyl; and
R₄ is a bridged cycloalkenyl ring (e.g. cyclohexene ring with an alkyl bridge such as a methylene bridge which may be optionally substituted, for example optionally substituted with a cycloalkyl ring such as a C₃₋₆ cycloalkyl ring (e.g. a cyclopropane ring)). The alkyl moiety may, for example, be a C₁₋₃ alkyl chain. The cycloalkyl moiety may, for example, be a C₅₋₇ ring.

In certain embodiments, R₁ and R₂ are each independently selected from H, Cl, F, Br, CH₃, CF₃, SH, -N(C₁₋₃alkyl)₂, -NHC(O)C₁₋₃alkyl, and -NHC(O)C₅₋₇cycloalkyl.

In certain embodiments, R₃ is H or C₁₋₃ alkyl.

In certain embodiments, R₄ is 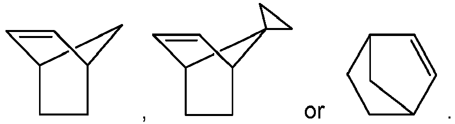 R₄ may be bonded to the adjoining atoms in the compound of formula (I) at the positions indicated by the dashed lines,

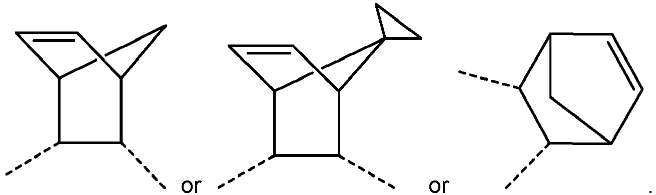

In certain embodiments, R₃ is H.

In certain embodiments, R₁ and R₂ are each individually selected from H, Cl, Br and - NHC(O)CH₃, R₃ is H, and R₄ is

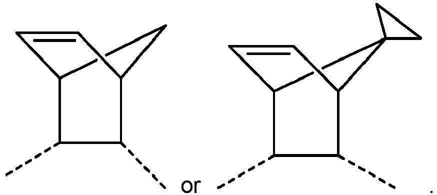

In certain embodiments, R₁ and R₂ are each individually selected from H, Cl, CH₃, - NHC(O)CH₃, -NHC(O)cyclohexane, or -N(CH₃)₂, R₃ is H and R₄ is

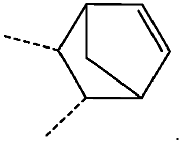

The compound according to formula (I) may, for example, be 3-(N-(3,4-dichlorophenyl)carbamoyl)-5-norbornene-2-carboxylic acid, also named 3-{[(3,4-dichlorophenyl)amino]carbonyl}bicyclo[2.2.1]hept-5-ene-2-carboxylic acid, known as CADD522 (CAS No: 199735-88-1), 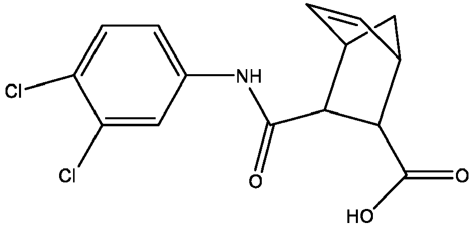 or a salt, ester or prodrug thereof, preferably a pharmaceutically acceptable salt, ester or prodrug thereof.

Alternatively, the compound according to formula (I) may, for example, be one or more selected from the following: 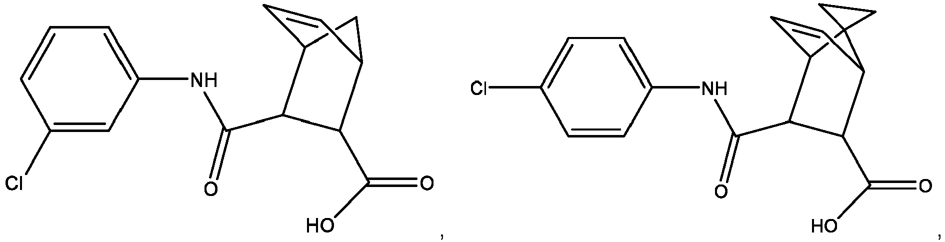 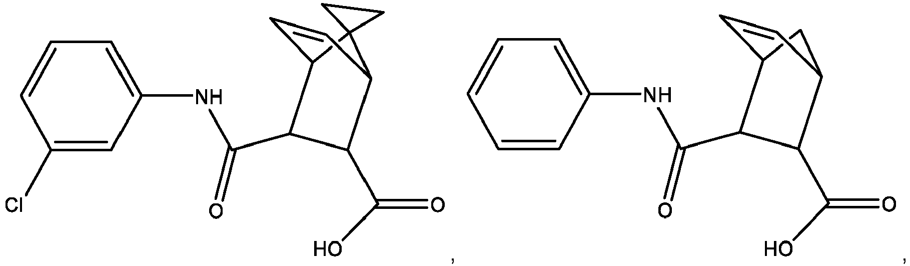 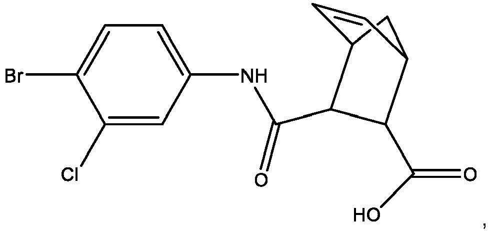 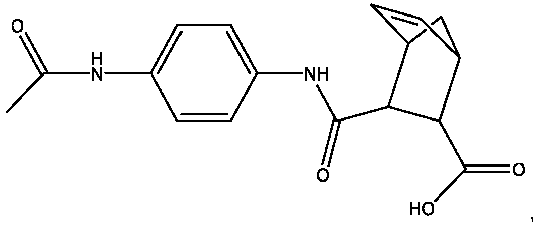 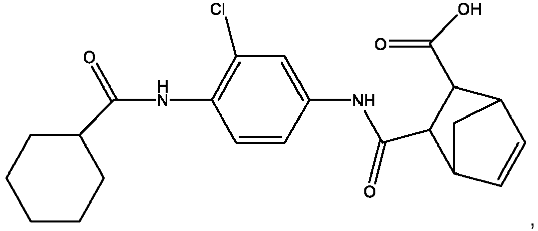 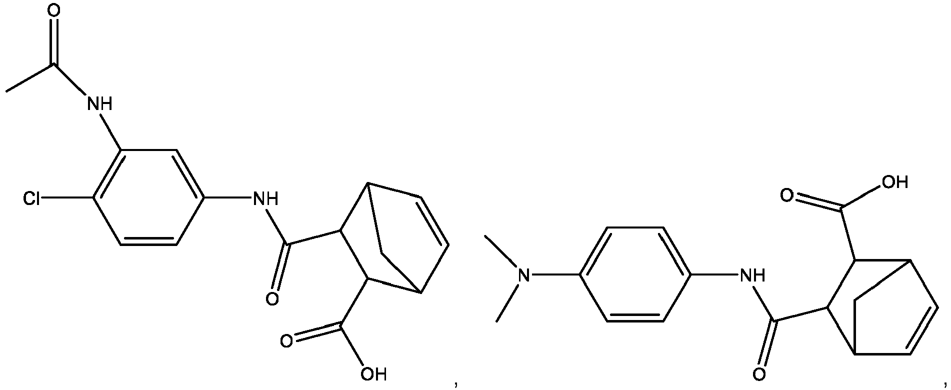 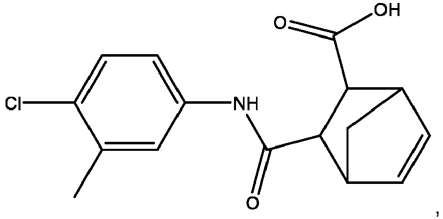 and salts, esters and prodrugs thereof, for example pharmaceutically acceptable salts, esters and prodrugs thereof.

In one particular embodiment of the present invention, the said compound of formula (I) or a salt, ester or prodrug thereof, for example a pharmaceutically acceptable salt, ester or prodrug thereof serves *in vivo* to inhibit (preferably specifically and/or selectively inhibit) the binding of RUNX2 to DNA. This inhibition of the binding of RUNX2 to DNA particularly takes place in the tumour cells in the *in vivo* treatment. Where the agent is a prodrug of a compound of formula (I) or a prodrug of a salt or ester thereof, for example a prodrug of a pharmaceutically acceptable salt or ester thereof, the prodrug is selected to be converted by the patient's *in vivo* metabolism to an active agent of formula (I) or a prodrug of a salt or ester thereof, for example a prodrug of a pharmaceutically acceptable salt or ester thereof after administration, as will be familiar to pharmaceutical chemists having knowledge of prodrug design.

The inhibitor of YBX1 activity or the inhibitor of YBX1 expression may, for example, be a small RNA (sRNA) molecule comprising a SCUBYC motif. The term "small RNA molecule" refers to an RNA molecule comprising less than about 35 nucleotides, for example comprising from about 14 to about 35 nucleotides, for example comprising from about 19 to about 35 nucleotides, for example comprising from about 19 to about 33 nucleotides. Small RNA molecules may be single or double stranded and include, for example, miRNAs, piRNAs, siRNAs, tRNAs, tRNA fragments (tRFs), molecules that mimic endogenous RNA molecules, and combinations of one or more thereof.

The SCUBYC recognition motif is shown in Figure 1(c). It comprises, in the following order, a first nucleotide that is C or G, a second nucleotide that is C, a third nucleotide that is U, a fourth nucleotide that is C, G or U, a fifth nucleotide that is C or U and a sixth nucleotide that is C. This motif is a binding site for YBX1.

The inhibitor of YBX1 activity or the inhibitor of YBX1 expression may, for example, be a tRNA derived fragment (tRF) having a SCUBYC motif or an analogue thereof. For example, the inhibitor of YBX1 activity or the inhibitor of YBX1 expression may be tRF-Gly-TCC or an analogue thereof. For example, the inhibitor of YBX1 activity or the inhibitor of YBX1 expression may be tRF-Lys-TTT or an analogue thereof.

The term "analogue" refers to a molecule that has the same activity as the original molecule but has a different structure. Since the same activity is required, the difference in structure is usually minor. For example, an analogue to a tRF having a SCUBYC motif would include the SCUBYC motif and therefore maintain its ability to bind YBX1, but the other nucleotides in the tRF that are not essential for YBX1 binding may be different to the original tRF.

The compositions used in the methods of treatment described but not claimed herein may, for example, be pharmaceutical compositions. The term "pharmaceutical composition" refers to a composition comprising (a pharmaceutically effective amount of) a therapeutic active agent (i.e. the one or more of an inhibitor of RUNX2 activity, an inhibitor of RUNX2 expression, an inhibitor of YBX1 activity and an inhibitor of YBX1 expression). The pharmaceutical compositions disclosed herein may additionally comprise one or more pharmaceutically acceptable carriers and/or excipients and/or diluents. The phrase "pharmaceutically acceptable" refers to compositions that do not produce an adverse, allergic or other untoward reaction when administered to an animal, such as, for example, a human.

The compositions and pharmaceutical compositions disclosed herein may further contain ingredients selected from, for example, adjuvants, carriers (solvents) such as water, ethanol, polyols (e.g. glycerol, propylene glycol, liquid polyethylene glycol), lipids, and combinations thereof, preserving agents, stabilisers, fillers, binders, disintegrating agents, wetting agents, emulsifying agents, suspending agents, sweetening agents, flavouring agents, perfuming agents, lubricating agents, coating agents, encapsulating agents and dispersing agents, depending on the nature of the mode of administration and dosage forms.

The compositions and pharmaceutical compositions disclosed herein may take the form, for example, of a solid preparation including tablets, capsules, caplets, drageés, lozenges, granules, powders, pellets, beads, cachets and bolus; and a liquid preparations including elixir, syrups, suspension, spray, emulsion, lotion, solution or tincture; or a semi-solid preparation including ointment, cream, paste, gel or jelly. Also included are solid form preparations, for example, tablets, capsules, granules and powder, which are intended to be converted, shortly before use, to liquid form preparations for oral administration. Techniques and formulations generally may be found in Remington, The Science and Practice of Pharmacy, Mack Publishing Co., Easton, PA, latest edition.

Any suitable mode of administration may be used. For example, the administration may be oral, local, parenteral (including intravenous, intramuscular, subcutaneous and intradermal), transdermal (including percutaneous) or transmucosal (including inhalation, nasal and sublingual). The administration may be sufficient to contact the tumour cells with the inhibitor of RUNX2 activity or the inhibitor of RUNX2 expression or the inhibitor of YBX1 activity or the inhibitor of YBX1 expression.

The composition may, for example, be administered to the subject for any period of time suitable to obtain a desired result (e.g. treatment of the cartilage matrix-forming tumour or metastatic cancer originating from cartilage matrix-forming tumour). The composition may, for example, be administered at any frequency suitable to obtain the desired result, for example daily or weekly or monthly. This includes intermittent and/or repeated administration.

The amount of composition administered may be varied depending on the subject, the severity of the disease to be treated and exact identity of the active agent. Determination of the proper amount/dosage for a particular situation is within the skill of the art. For example, for therapeutic applications a physician or veterinarian having ordinary skill in the art can readily determine and prescribe the effective amount of the composition required. The total daily amount/dosage may be divided and administered in portions during the day if desired.

In general, a suitable dose of active agents will be that amount which is the lowest dose effective to produce the desired effect. A person of ordinary skill in the art will understand that a suitable dose or dosage will typically vary from subject to subject, and will dependent on factors such as the severity of health conditions of the subject at the outset of administration of the composition.

The inhibitor of RUNX2 activity or the inhibitor of RUNX2 expression or the inhibitor of YBX1 activity or the inhibitor of YBX1 expression may be administered in combination with another therapeutic agent, for example another therapeutic agent against the cartilage matrix-forming tumour or metastatic cancer originating from a cartilage matrix-forming tumour and/or another therapeutic agent against any side effects of the treatment, for example side effects that can affect therapeutic efficacy.

### Methods of Diagnosis and Prognosis

The present invention is further based on the surprising finding that the expression level of RLINX2 and YBX1 in the cartilage matrix-forming bone cancer, chondrosarcoma, is higher than the respective expression level of RUNX2 and YBX1 in normal cartilage or other biological material, and that the expression level of RUNX2 and YBX1 is higher in high grade tumours compared to the respective expression level in low or intermediate grade tumours.

There is therefore also provided but not claimed herein *in vitro* methods of diagnosing the presence of or risk of developing a cartilage matrix-forming bone tumour and of determining the prognosis of a subject having a cartilage matrix-forming bone tumour. The methods comprise (i) measuring the expression level of at least one of RUNX2 and YBX1 in a biological sample obtained from the subject, and (ii) comparing the expression level of RUNX2 and/or YBX1 in the biological sample obtained from the subject with the respective expression level of RUNX2 and/or YBX1 in normal cartilage or other biological material. A higher expression level of RUNX2 and/or YBX1 in the biological sample obtained from the subject compared to the respective expression level in the normal cartilage or other biological material indicates the presence of or an increased risk of developing a cartilage matrix-forming bone tumour. The subject's prognosis decreases with increasing expression level of RUNX2 and/or YBX1 in the biological sample obtained from the subject. The expression level of one of RUNX2 and YBX1 may be measured. The expression level of both of RUNX2 and YBX1 may be measured.

A decreasing prognosis means that the severity of the disease is worse, for example the tumour is more aggressive and/or has a greater potential for metastatic activity. The subject's chance of survival therefore decreases with decreasing prognosis. The methods of prognosis described herein may assist a clinician in monitoring the progress of the cartilage matrix-forming bone tumour over time and may assist the clinician in determining a proper course of treatment of the cartilage matrix-forming bone tumour.

World Health Organization (WHO) guidelines can be used to classify cartilage matrix-forming bone cancers as grade one (low grade), grade two (intermediate grade) or grade three (high grade) (World Health Organization, 2013, "Classification of Tumours of Soft Tissue and Bone", 4th Edition, Volume 5). WHO guidelines also outline benign tumours.

The biological sample obtained from the subject and/or a sample of normal cartilage or other normal biological material may, for example, be a tissue, cells or fluid isolated from the subject or other individual, as the case may be. The sample may, for example, be a tissue resection or biopsy, for example a tissue resection or biopsy from the bone or cartilage or tumour, other tissue resections or biopsies such as from the skin, the lymph node or the respiratory, intestinal or genitourinary tracts. Alternatively, the sample may, for example, be blood, plasma, serum, tumour biopsy, urine, saliva, stool, sputum, spinal fluid, pleural fluid, nipple aspirates or lymph fluid. The sample may, for example, be tumour cells isolated from a biological sample such as tumour cells isolated from blood. The biological sample may, for example, be treated before the amount of RUNX2 or YBX1 is determined to prevent degradation of protein and/or RNA.

The sample(s) used in the methods for detecting the presence of or risk of developing a cartilage matrix-forming bone tumour described herein may, for example, be a resection or biopsy from bone tissue or cartilage tissue or tumour.

The sample(s) used in the methods for determining the prognosis of a subject having a cartilage matrix-forming bone tumour may, for example, be a tumour biopsy or resection, for example a tumour biopsy or resection from the primary tumour.

The expression level of RUNX2, YBX1 and any other gene or molecule may, for example, be determined by any method known to those skilled in the art. For example, expression level may be determined by measuring the amount of the protein, and/or amount of mRNA, and/or amount of tRNA, and/or amount of tRF, and/or amount of miRNA.

The amount of protein and/or mRNA and/or tRNA and/or tRF and/or miRNA may be determined using a probe specific to that protein, mRNA, tRNA, tRF or miRNA. The probe may, for example, be labelled (e.g. fluorescently labelled, radioactively labelled, chemiluminescently labelled, or enzymatically labelled). The probe may, for example, be labelled with a tag such as a myc tag, HA tag, VSV-G tag, HSV tag, FLAG tag, V5 tag or HIS tag. The probe may, for example, be an antibody. The amount of protein, mRNA, tRNA, tRF or miRNA may be correlated to the intensity of the signal emitted from the labelled probe. The probe may, for example, be a DNA or RNA probe. The protein or RNA may be isolated from the biological sample and optionally amplified before contacting with the probe. Alternatively, the probe may be contacted with the biological sample itself.

The amount of protein or RNA may be measured by methods that are well-known to persons skilled in the art such as, for example, Western blotting and Northern blotting. Known amplification methods such as reverse transcription and/or polymerase chain reaction (PCR) may also be used. DNA/RNA arrays, microarrays and chips may be used. Immunoassays such as enzyme linked immunoabsorbent assays (ELISA), radioimmunoassay (RIA) and immunoradiometric assay (IRMA) may also be used. Aptamer technology may also be used.

The amount of protein or RNA may also be determined using mass spectrometry. The mass spectrometry may, for example, be MALDI/TOF (time of flight), SEL-DI/TOF, liquid chromatography (LC-MS), gas chromatography (GC-MS), high performance liquid chromatography (LPLC-MS), capillary electrophoresis (CE-MS), nuclear magnetic resonance (NMR-MS) or tandem mass spectrometry.

The amount of a particular protein or RNA in the biological sample obtained from the subject and in the normal cartilage or other biological material should be normalized to a loading control. The loading control is a protein or RNA with high and ubiquitous expression. Examples of loading controls include, for example, U6, actin, GAPDH, tubulin, VDCA1, histone H2B, PCNA and TBP. The normalized amount of the protein or RNA in the biological sample can then be compared to the normalized amount of the protein or RNA in the normal cartilage or other biological material.

A higher expression level in the biological sample obtained from the subject may, for example, refer to a level of occurrence that is statistically significantly above the level in the normal cartilage or other (normal) biological material. For example, the statistically significant higher expression level may be 2 standard deviation above the expression level in normal cartilage tissue or other biological material.

The presence of or increased risk of developing a cartilage matrix-forming bone tumour may, for example, be indicated by a level of RUNX2 and/or YBX1 in a biological sample obtained from a subject that is at least about 1.5 times higher than the level in normal cartilage or other normal biological material. For example, the presence of or an increased risk of developing a cartilage matrix-forming bone tumour may be indicated by a level of RUNX2 and/or YBX1 in a biological sample obtained from a subject that is at least about 2 time higher or at least about 2.5 times higher or at least about 3 times higher or at least about 3.5 times higher or at least about 4 times higher or at least about 4.5 times higher or at least about 5 times higher than the level in normal cartilage or other normal biological material.

A level of RUNX2 and/or YBX1 in a biological sample obtained from a subject that is at least about 16 times greater than the respective level in normal cartilage or other normal biological material may be indicative of a high grade tumour. For example, a level of RUNX2 and/or YBX1 in a biological sample obtained from a subject that is at least about 18 or at least about 20 or at least about 22 or at least about 24 or at least about 25 or at least about 26 or at least about 28 or at least about 30 or at least about 32 or at least about 34 or at least about 35 or at least about 36 or at least about 38 or at least about 40 times greater than the respective level in normal cartilage or other normal biological material may be indicative of a high grade tumour.

A level of RUNX2 and/or YBX1 in a biological sample obtained from a subject that is from about 10 to about 15 times greater than the respective level in normal cartilage or other normal biological material may be indicative of an intermediate grade tumour.
A level of RUNX2 and/or YBX1 in a biological sample obtained from a subject that is from about 1.5 to about 9 times greater than the respective level in normal cartilage or other normal biological material may be indicative of a low grade tumour. For example, a level of RUNX2 and/or YBX1 in a biological sample obtained from a subject that is from about 2 to about 9 or from about 2.5 to about 9 or from about 3 to about 9 or from about 3.5 to about 9 or from about 4 to about 9 or from about 4.5 to about 9 or from about 5 to about 9 times greater than the respective level in normal cartilage or other normal biological material may be indicative of a low grade tumour.

The methods described herein may further comprise measuring the expression level of one or more further markers that are indicative of the presence of or increased risk of developing a cartilage matrix-forming bone tumour, or may be indicative of the prognosis of a subject having a cartilage matrix-forming bone tumour.

The methods described herein may further comprise measuring the expression level of one or more tRF having a SCUBYC motif in the biological sample obtained from the subject. For example, the methods described herein may further comprise measuring the expression level of tRF-Gly-TCC and/or tRF-Lys-TTT in the biological sample obtained from the subject. A higher or lower expression level of the one or more tRF having a SCUBYC motif in the biological sample obtained from the subject compared to the expression level in normal cartilage or other biological material indicates the presence of or an increased risk of developing a cartilage matrix-forming bone tumour. For example, a level of the one or more tRF having a SCUBYC motif that is that is at least about 1.5 times higher or lower than the level in normal cartilage or other normal biological material may indicate the presence of or an increased risk of developing a cartilage matrix-forming bone tumour. For example, a level of the one or more tRF having a SCUBYC motif that is that is at least about 2 or at least about 2.5 or at least about 3 or at least about 3.5 or at least about 4 or at least about 4.5 or at least about 5 times higher or lower than the level in normal cartilage or other normal biological material may indicate the presence of or an increased risk of developing a cartilage matrix-forming bone tumour.

The methods described herein may further comprise measuring the expression level of one or more tRNA^{GlyTCC}, tRNA^{LysTTT}, miR-140, SOX9, PTHLP, ANG, HDAC4, and p53 in the biological sample obtained from the subject. The methods described herein may further comprise measuring the expression level of one or more tRNA^{GlyTCC}, tRNA^{LysTTT}, miR-140, SOX9, PTHLP, ANG, and p53 in the biological sample obtained from the subject. The methods described herein may further comprise measuring the expression level of two, three, four five, six or seven of tRNA^{GlyTCC}, tRNA^{LysTTT}, miR-140, SOX9, PTHLP, ANG, HDAC4, and p53 in the biological sample obtained from the subject. In particular, the methods described herein may further comprise measuring the expression level of PTHLP, and optionally one or more of tRNA^{GlyTCC}, tRNA^{LysTTT}, miR-140, SOX9, ANG, HDAC4, and p53. A higher expression level of tRNA^{GlyTCC}, tRNA^{LysTTT}, miR-140, and p53 in the biological sample obtained from the subject compared to the expression level in normal cartilage or other biological material indicates the presence of or an increased risk of developing a cartilage matrix-forming bone tumour. A lower expression level of SOX9, PTHLP and ANG in the biological sample obtained from the subject compared to the expression level in normal cartilage or other biological material indicates the presence of or an increased risk of developing a cartilage matrix-forming bone tumour. A higher or lower expression level of HDAC4 in the biological sample obtained from the subject compared to the expression level in normal cartilage or other biological material indicates the presence of or an increased risk of developing a cartilage matrix-forming bone tumour. In particular, a lower expression level of HDAC4 in the biological sample obtained from the subject compared to the expression level in normal cartilage or other biological material indicates the presence of or an increased risk of developing a low grade cartilage matrix-forming bone tumour. A higher expression level of HDAC4 in the biological sample obtained from the subject compared to the expression level in normal cartilage or other biological material indicates the presence of or an increased risk of developing an intermediate or high grade cartilage matrix-forming bone tumour.

For example, a level of the one or more of tRNA^{GlyTCC}, tRNA^{LysTTT}, miR-140, HDAC4, and p53 that is that is at least about 1.5 times higher than the level in normal cartilage or other normal biological material may indicate the presence of or an increased risk of developing a cartilage matrix-forming bone tumour. For example, a level of the one or more of SOX9, PTHLP, HDAC4, and ANG that is that is at least about 1.5 times lower than the level in normal cartilage or other normal biological material may indicate the presence of or an increased risk of developing a cartilage matrix-forming bone tumour. For example, a level of the one or more of tRNA^{GlyTCC}, tRNA^{LysTTT}, miR-140, HDAC4, and p53 that is that is at least about 2 or at least about 2.5 or at least about 3 or at least about 3.5 or at least about 4 or at least about 4.5 or at least about 5 times higher than the level in normal cartilage or other normal biological material may indicate the presence of or an increased risk of developing a cartilage matrix-forming bone tumour. For example, a level of the one or more of SOX9, PTHLP, HDAC4, and ANG that is that is at least about 2 or at least about 2.5 or at least about 3 or at least about 3.5 or at least about 4 or at least about 4.5 or at least about 5 times lower than the level in normal cartilage or other normal biological material may indicate the presence of or an increased risk of developing a cartilage matrix-forming bone tumour.

In the methods of diagnosis described herein, a second confirmatory diagnosis step may take place, such as, for example, biopsy, ultrasound, PET scanning, MRI or any other imaging technique.

The methods of diagnosis and/or prognosis described herein may, for example, further comprise a step of treating a subject having a cartilage matrix-forming bone tumour and/or a metastatic cancer originating from a cartilage matrix-forming bone tumour. The method of treatment may, for example, be in accordance with the methods of treatment described herein, for example wherein one or more of an inhibitor of RUNX2 activity, an inhibitor of RUNX2 expression, an inhibitor of YBX1 activity and an inhibitor of YBX1 expression is administered to the subject in need thereof. Alternatively or additionally, the method of treatment may comprise any method for treating a subject having a cartilage matrix-forming bone tumour and/or metastatic cancer originating from a cartilage matrix-forming bone tumour that is known in the art such as, for example, curettage, resection and/or amputation.

There is therefore provided but not claimed herein a method of treating a cartilage matrix-forming bone tumour and/or a metastatic cancer originating from a cartilage matrix-forming bone tumour in a subject, the method comprising obtaining or receiving the results of an assay that measures the expression level of at least one of RUNX2 and YBX1 in a subject, for example in a biological sample obtained from the subject, and, if the level of RUNX2 and/or YBX1 is higher than a reference level, thereby indicating that the subject has or is at risk of developing a cartilage matrix-forming bone tumour and/or a metastatic cancer originating from a cartilage matrix-forming bone tumour, treating the cartilage matrix-forming bone tumour and/or the metastatic cancer originating from a cartilage matrix-forming bone tumour, for example by administering one or more therapeutic agents such as one or more of an inhibitor of RUNX2 activity, an inhibitor of RUNX2 expression, an inhibitor of YBX1 activity and an inhibitor of YBX1, or for example by curettage, resection and/or amputation. The reference level may, for example, the expression level of RUNX2 and/or YBX1 in normal cartilage or other biological material.

The results of an assay that measures the expression level of at least one of RUNX2 and YBX1 in a subject may, for example, be obtained by or have been obtained by one of the methods described herein. For example, the results of an assay that measures the expression level of at least one of RUNX2 and YBX1 in a subject may, for example, be obtained by or have been obtained by (i) measuring the expression level of at least one of RUNX2 and YBX1 in a biological sample obtained from the subject, and (ii) comparing the expression level of RUNX2 and/or YBX1 in the biological sample obtained from the subject with the respective expression level of RUNX2 and/or YBX1 in normal cartilage or other biological material.

There is further provided but not claimed herein methods for performing an assay, the method comprising measuring the expression level of at least one of RUNX2 and YBX1 in a biological sample obtained from the subject. The method may further comprise comparing the expression level of RUNX2 and/or YBX1 in the biological sample obtained from the subject with the respective expression level of RUNX2 and/or YBX1 in normal cartilage or other biological material.

The invention and disclosure will now be described in detail by way of reference only to the following non-limiting examples.

### EXAMPLES

### MATERIALS AND METHODS

### Patient samples

This study included 13 chondrosarcoma patients (ages 27-75) who underwent surgery at The Royal Orthopaedic Hospital, Birmingham, from 2014 to 2016. Primary tumours were collected following surgery with gross pathology and grade determined at the time of removal. We used the WHO guidelines to classify grade one conventional chondrosarcoma as "low grade". Grade two conventional chondrosarcoma was classified as "intermediate grade". Dedifferentiated chondrosarcoma was classified as "high grade". This study also included 6 control patients (ages 58-93) who underwent surgery at the Norfolk and Norwich University Hospital following a neck of femur fracture. Cartilage matrix was collected following surgery. Exclusion criteria for control samples was the presence of inflammatory or autoimmune disease. The FMH Research Ethics Committee approved the collection and study of patient material (reference 2013/2014 - 22 HT). All individuals provided written informed consent to donate tissue to this study.

### Cell culture

SW1353 (chondrosarcoma) cells were cultured in 1:1 DMEM/F-12 Supplement (Thermo Fisher Scientific) containing 10% (volume/volume) fetal bovine serum (Sigma Aldrich). Cells were fed with culture medium every other day and maintained at 37°C in a hypoxic atmosphere of 5% CO₂. Cells were obtained from ATCC and authenticated by STR profiling. Cells were regularly monitored for *Mycoplasma* infection by PCR. Phenotypic checks for interleukin 1 response ensured cell phenotype was maintained.

For the experiment reported in Figure 8(b) the culture medium additionally contained 1% (v/v) penicillin streptomycin.

### Next generation sequencing

Tissue samples were homogenised under liquid nitrogen conditions using the BioPulverisor (BioSpec). Total RNA was extracted using the miRCURY RNA isolation kit (Exiqon) according to manufacturer's instructions. RNA concentration and integrity was measured on the NanoDrop 8000 Spectrophotometer (Thermo Fisher Scientific) and visually assessed by agarose gel electrophoresis with ethidium bromide staining. RNA was stored at -80°C. All 19 libraries were constructed using 1 µg of RNA that was ligated to 3' and 5' HD adapters (Xu, P. et al. An improved protocol for small RNA library construction using high definition adapters. Methods in Next Generation Sequencing 2, 1-10 (2015). Ligated RNA products were reverse transcribed to cDNA and amplified by PCR. The cDNA products expected to contain 19-33 base pair inserts were purified by 8% polyacrylamide gel electrophoresis and ethanol precipitation. We performed 50 bp single end sequencing on the HiSeq 2500 Ultra High Throughput Sequencing System (Illumina).

### Bioinformatics

Raw fastq files were converted to fasta format. Reads containing unassigned nucleotides were excluded. The 3' adapter was trimmed using perfect sequence match to the first 8 nucleotides of the 3' HiSeq 2500 adapter (TGGAATTC). The HD signatures (four assigned nucleotides at the ligating ends) of the reads were also trimmed (Beckers, M.L. et al. Comprehensive processing of high throughput small RNA sequencing data including quality checking, normalization and differential expression analysis using the UEA sRNA Workbench. Rna (2017)). Reads longer than 17 nt were kept for further analysis. Reads with low sequence complexity, i.e. with an overrepresentation of one nucleotide for more than 60% of the sequence length, were excluded. As part of the quality check the size class distributions for redundant reads, i.e. total reads (with their abundance) and non-redundant reads, i.e. unique reads (with their abundance), were plotted side by side with the complexity, which is defined as the ratio of non-redundant to redundant reads (Mohorianu, I. et al. Profiling of short RNAs during fleshy fruit development reveals stage-specific sRNAome expression patterns. Plant J 67, 232-46 (2011)). SRNAs were mapped full length with no gaps or mismatches allowed to the human genome (v.38) and corresponding annotations using PatMaN (Prufer, K. et al. PatMaN: rapid alignment of short sequences to large databases. Bioinformatics 24, 1530-1 (2008)). The latest set of human miRNAs was downloaded from miRBase v.21 (Kozomara, A. & Griffiths-Jones, S. miRBase: annotating high confidence microRNAs using deep sequencing data. Nucleic Acids Res 42, D68-73 (2014)). SRNA expression levels were normalised using a scaling approach, reads per total, to a fixed total of 10 million reads (Mortazavi, A., Williams, B.A., McCue, K., Schaeffer, L. & Wold, B. Mapping and quantifying mammalian transcriptomes by RNA-Seq. Nat Methods 5, 621-8 (2008)). Differentially expressed reads were identified using both the localisation of maximal expression intervals for the control versus cancer comparisons and pairwise comparisons using offset fold change with an empirically determined offset of 20 (Mohorianu, I. et al. Profiling of short RNAs during fleshy fruit development reveals stage-specific sRNAome expression patterns. Plant J 67, 232-46 (2011) and Mohorianu, I., Stocks, M.B., Wood, J., Dalmay, T. & Moulton, V. CoLlde: a bioinformatics tool for CO-expression-based small RNA Loci Identification using high-throughput sequencing data. RNA Biol 10, 1221-30 (2013)).

### Northern blot

We loaded 1 µg of RNA mixed with Ambion gel loading buffer II (Thermo Fisher Scientific) to a 16% polyacrylamide gel with urea. The gel was run at 120 V for 2 hours in 0.5X TBE. We transferred the RNA to a Hybond-NX (GE Healthcare) membrane using semidry transfer conditions at 250 mA for 45 minutes. We crosslinked the RNA to the membrane by adding 5 mL crosslinking solution adjusted to pH 8 (12 mL water, 122.5 mL 12.5 M 1-methylimidazole, 10 mL 1 M hydrochloric acid and 0.373 g of 1-ethyl-3-(3-dimethylaminopropyl) carbodiimide) and incubating at 60°C for 1 hour in saran wrap. For each sRNA we pre-hybridised the membrane with ultra-hyb-oligo buffer (Thermo Fisher Scientific) at 37°C for 1 hour. We then incubated a mixture of 10 µL water, 4 µL 5X polynucleotide kinase forward buffer (New England Biolabs), 2 µL 10 mM DNA antisense oligonucleotide, 1 µL T4 polynucleotide kinase (New England Biolabs) and 3 µL γ-ATP at 37°C for 1 hour. We incubated the membrane in this buffer rotating overnight at 37°C and then washed it three times in 0.2X SSC, 0.1% SDS before exposing it on a phosphorimaging screen in a radioactive cassette (Fujifilm) followed by imaging on the FX Pro Plus molecular imager (Bio-Rad). The same membrane was re-probed using antisense DNA oligonucleotides (Sigma Aldrich) which were generated using the sequences shown in Table 1 below. We used U6 as a loading control.

**Table 1.**

| **sRNA** | **Sequence type** | **Sequence (5'-3')** |
|---|---|---|
| Negative control 1 | RNA mimic | GAUGGCAUUCGUCAGUUCUA |
| Negative control 2 | LNA inhibitor | TAACACGTCTATACGCCCA |
| tRF-Gly-TCC | RNA mimic | GCGUUGGUGGUAUAGUGGUGAGCAUAGC UGCC |
| tRF-Lys-TTT | RNA mimic | GCCCGGAUAGCUCAGUCGGUAGAGCAUC AGACU |
| tRF-Asn-GTT | RNA mimic | UUGGUGGUUCGAGCCCACCCAGGGACG |
| tRF-Gly-TCC | LNA inhibitor | TATGCTCACCACTATACCAC |
| tRF-Lys-TTT | LNA inhibitor | CTCTACCGACTGAGCTATCC |
| tRF-Asn-GTT | LNA inhibitor | GTGGGCTCGAACCACCAA |
| miR-140-3p | LNA mimic | TACCACAGGGTAGAACCACGG |
| miR-320a | LNA mimic | AAAAGCTGGGTTGAGAGGGCGA |
| miR-486-5p | LNA mimic | TCCTGTACTGAGCTGCCCCGAG |
| miR-140-3p | LNA inhibitor | CGTGGTTCTACCCTGTGGT |
| miR-320a | LNA inhibitor | CGCCCTCTCAACCCAGCTTT |
| miR-486-5p | LNA inhibitor | TCGGGGCAGCTCAGTACAG |

### Western blot

Protein was extracted with M-PER and T-PER (Pierce) for 30 minutes on ice in the presence of HALT protease inhibitor cocktail (Pierce). Samples were clarified by centrifugation (10,600 x g for 10 minutes). Using the supernatant, protein concentrations were determined using the BCA protein assay kit (Pierce) according to the manufacturer's protocols. Equal amount of proteins were separated on 4-12% SDS-PAGE gradient gels. Gels were analysed by blotting onto immobilon PVDF (Millipore) membranes using antibodies to YBX1 (Abcam). Antibodies were produced in rabbits immunised with a synthetic peptide conjugated to KLH derived from residues 1-100 of human YBX1 as previously described (Eliscovich, C., Shenoy, S.M. & Singer, R.H. Imaging mRNA and protein interactions within neurons. Proc Natl Acad Sci U S A 114, E1875-e1884 (2017)). Primary antibodies were detected using IRDye labelled secondary antibodies (Li-Cor Biosciences) at 1:10,000 dilution. YBX1 was visualised using the CLx infrared imaging system (Odyssey).

### Tumour sphere assay

Tumour spheres were constructed as previously described (Greco, K.V. et al. High density micromass cultures of a human chondrocyte cell line: a reliable assay system to reveal the modulatory functions of pharmacological agents. Biochem Pharmacol 82, 1919-29 (2011)). Briefly, 80% confluent monolayer cell cultures were released by trypsin-EDTA and resuspended in growth medium at a density of 4.1 × 10⁴ cells/mL. Tumour spheres were achieved by pipetting 20 µL of cell suspension into individual wells of 24 well plates. Following a 3 hour intermolecular cohesion period 500 µL of growth medium was gently added. Cultures formed three dimensional spheres over the next 24 hours.

### LNA and RNA transfection

Once tumour spheres had formed, tRF mimics (RNA), tRF inhibitors (LNA), miRNA mimics (LNA) and miRNA inhibitors (LNA) (Exiqon) were transfected using Lipofectamine 3000 (Thermo Fisher Scientific) in reduced serum media (Thermo Fisher Scientific) for a final concentration of 50 nM consisting of equal parts of each mimic or inhibitor (sequences in Table 1 above). Confirmation of cell entry at 50 nM concentration was determined after performing the transfection with FAM labelled sequences. After 6 hours of incubation reduced serum media containing mimics or inhibitors was replaced with fresh growth media. Tumour spheres were subjected to histology, fluorescence microscopy and transcriptional profiling 48 hours later. Transfections were performed in triplicate in 3 independent experiments.

### Histology and fluorescence microscopy

Tumour spheres were fixed in 3.7% formaldehyde and treated with 0.2% Triton-X 100. Nuclear 4', 6-diamidino-2-phenylindole (DAPI) and Texas Red-X Phalloidin (F-actin) staining were imaged using a Zeiss Axiovert 200M microscope with an Axiocam MRm CCD camera under the control of AxioVision software. DAPI fluorescence was excited at 365 nm and emission collected between 420 and 470 nm. Texas Red-X Phalloidin fluorescence was excited at 558 nm and emission collected through a 615 nm LP filter. Nuclear DAPI staining was segmented with the number and size of the nuclei quantified using imaged.

### Transcriptional profiling

We used qPCR as previously described (Fricke, C., Green, D., Smith, D., Dalmay, T. & Chapman, T. MicroRNAs influence reproductive responses by females to male sex peptide in Drosophila melanogaster. Genetics 198, 1603-19 (2014)). Due to a limited amount of RNA, qPCR was performed on pools of control tissue, low grade, intermediate grade and high grade tumours. We also extracted and pooled RNA from 6 replicates of tumour spheres treated with a tRF-Gly-TCC mimic. We used Taqman gene expression assays for *SOX9, HDAC4, RUNX2, PTHLH, YBX1, ANG, IHH* and *TP53* (Thermo Fisher Scientific) following the manufacturer's protocol. We used *ACTB* as a reference to normalise gene expression. QPCR was performed in a final volume of 10 µL using a 7500 Fast Real Time PCR instrument (Applied Biosystems) under the following cycling conditions: 95 °C for 20 seconds, 40 cycles at 95 °C for 3 seconds and 60 °C for 30 seconds. QPCR was performed in triplicate and data is shown as mean ± SEM.

### Statistical analysis

Evaluation of variability between sequencing libraries was conducted using scatter plots, size-split boxplots of the replicate-to-replicate differential expression, intersection and Jaccard similarity analyses (Mohorianu, I. et al. Profiling of short RNAs during fleshy fruit development reveals stage-specific sRNAome expression patterns. Plant J 67, 232-46 (2011)). The empirical differential expression analysis was confirmed by parametric (t-tests) and non-parametric (Mann-Whiney-U) tests. For the statistical tests we considered p<0.05 as statistically significant. Analysis was conducted using Perl v5.22 and R v3.4.3.

### Small molecule treatment

Growth media on chondrosarcoma tumour spheres was replaced with fresh media containing 0.1% DMSO (control 1), fresh media containing 0.2% DMSO (control 2), fresh media containing 50 µM small molecule 3-(N-(3,4-dichlorophenyl)carbamoyl)-5-norbornene-2-carboxylic acid, also named 3-{[(3,4-dichlorophenyl)amino]carbonyl}bicyclo[2.2.1]hept-5-ene-2-carboxylic acid, known as CADD522, fresh media containing 100 µM small molecule (CADD522). Tumour spheres were incubated in this media for 72 hours. We fixed tumour spheres in 3.7% formaldehyde plus 0.2% Triton-X 100. We visualised DAPI staining using an Axiovert 200M microscope (Zeiss) with an Axiocam MRm CCD camera (Zeiss) under the control of AxioVision software. DAPI fluorescence was excited at 365 nm and emission collected between 420 and 470 nm. Nuclear DAPI staining was segmented with the number of the nuclei quantified using imaged. Experiment was performed in triplicate to achieve mean cell nuclei. Statistical significance (**p* = <0.002 and ***p* = <0.005) was calculated using an unpaired t test with Prism 6.

### RESULTS

### SRNA expression is modulated during chondrosarcoma progression

Sequencing reads matching to the human genome were normalised using the reads per million approach at 10 million reads per library. Matching against the human genome (v.38) revealed a bimodal size class distribution for the redundant reads with peaks at 22 and 32 nt. Further alignment against available annotations revealed these peaks correspond to miRNAs and tRFs. The presence of a small number of reads with high abundances is also supported by local minimums at 22 and 30-33 nt in the complexity distributions. As with all human studies there was variability in samples (when compared to inbred genetic models such as mice and fruit flies where variability is minimal) meaning differential expression was conducted using both a confidence interval approach and pairwise comparisons of normalised expression levels using offset fold change (Mohorianu, I., Stocks, M.B., Wood, J., Dalmay, T. & Moulton, V. CoLlde: a bioinformatics tool for CO-expression-based small RNA Loci Identification using high-throughput sequencing data. RNA Biol 10, 1221-30 (2013)). We found a significant differential expression, by at least four-fold, of 30 tRFs and 37 miRNAs during chondrosarcoma progression. We selected tRF-Gly-TCC, tRF-Lys-TTT, tRF-Asn-GTT (Figure 1A), miR-140, miR-320a and miR-486 for further analysis based on their expression level and relevance to sarcoma (Green, D., Dalmay, T. & Fraser, W.D. Role of miR-140 in embryonic bone development and cancer. Clin Sci (Lond) 129, 863-73 (2015), Green, D., Fraser, W.D. & Dalmay, T. Transfer RNA-derived small RNAs in the cancer transcriptome. Pflugers Arch (2016), Namlos, H.M. et al. Modulation of the osteosarcoma expression phenotype by microRNAs. PLoS One 7, e48086 (2012)). Sequence analysis of the tRFs revealed tRF-Gly-TCC contained a SCUBYC sequence motif at the 3' end (Figure 1B). This motif is known to interact with YBX1 (Figure 1C) (Goodarzi, H. et al. Endogenous tRNA-Derived Fragments Suppress Breast Cancer Progression via YBX1 Displacement. Cell 161, 790-802 (2015)). Normalised count read matrix showed that expression of tRF-Gly-TCC was steadily reduced in chondrosarcoma progression (Figure 1D).

### Expression of tRNA^{GlyTCC}, tRF-Gly-TCC, tRNA^{LysTTT}, tRF-Lys-TTT and miR-140 is modulated during chondrosarcoma progression

TRNAs and tRFs comprise different size classes. We carried out molecular validation of bioinformatics output by northern blot consisting of sample pools. For each selected tRNA and sRNA we observed negligible expression in control tissues except for tRF-Gly-TCC, which was visibly expressed (Figure 2A-E). In low grade tumours tRNA^{GlyTCC}, tRF-Gly-TCC, tRNA^{LysTTT} and tRF-Lys-TTT were expressed (Figure 2A-D). In intermediate grade tumours tRF-Gly-TCC and tRF-Lys-TTT were expressed (Figure 2B&D). In high grade tumours tRF-Gly-TCC expression was reduced compared to low and intermediate grade tumours (Figure 2B). In high grade tumours full length tRNA^{LysTTT}, tRF-Lys-TTT and miR-140 were highly expressed (Figure 2C-E). Consistent with their role in hypoxic stress response and breast cancer progression we find specific tRNAs and tRFs have a modulated expression in chondrosarcoma progression.

### MiR-140, a key driver of embryonic bone development, is highly expressed in high grade chondrosarcoma

We found that miR-140 is highly expressed in high grade chondrosarcoma (Figure 2E). We show a higher expression of miR-140 compared to controls, low grade and intermediate grade chondrosarcoma, suggesting restoration of miR-140-*HDAC4*-*RUNX2* embryonic bone development signalling is associated with high grade chondrosarcoma.

### YBX1 is upregulated in chondrosarcoma

As sequence analysis of tRF-Gly-TCC revealed a YBX1 recognition motif, tRF-Gly-TCC expression is attenuated from low grade to high grade and YBX1 can be oncogenic, we postulated that YBX1 is involved in regulating chondrosarcoma progression. We performed western blots to show a 47 kDa and a 30 kDa variant of YBX1 is expressed in a chondrosarcoma cell line (Figure 2H). A 27 kDa variant of YBX1 is highly expressed in primary tumours when compared to control (Figure 2I).

### Ectopic expression of tRF-Gly-TCC causes progression arrest in tumour spheres

We hypothesised that remodulating the plasticity of tRF and miRNA expression would have an effect on cellular/tumour phenotypes. Tumour spheres have emerged as promising experimental tools that reflect the natural state of cancer tissues better than traditional cell culture or non-human animal models (Shroyer, N.F. Tumor Organoids Fill the Niche. Cell Stem Cell 18, 686-7 (2016)). Tumour spheres are a biologically relevant system that will enable the discovery of novel therapeutics. We generated chondrosarcoma tumour spheres, induced overexpression and inhibition of tRF-Gly-TCC, tRF-Lys-TTT, tRF-Asn-GTT, miR-140, miR-320a and miR-496 using antisense locked nucleic acid (LNA) inhibitors and RNA mimics analogous to each sRNA. Nuclear and F-actin histology combined with inverted microscopy showed no major phenotypic effect except for the tRF-Gly-TCC mimic (Figure 3E). Though each mimic and inhibitor likely had an effect on gene expression, tumour spheres treated with the tRF-Gly-TCC mimic displayed a phenotype containing a low cancer cell number, shrunken nuclei and visible changes to morphology (Figure 3R&T). Brightfield imaging identified both a reduced cell number and smaller cell size in colonies treated with a tRF-Gly-TCC mimic (Figure 3T). The most significant change was a condensation of the nuclear DNA and a decrease in nuclear size shown by fluorescent staining of the DNA (Figure 3R). Image analysis showed a more than two-fold decrease in nuclear area of tumour spheres treated with a tRF-Gly-TCC mimic (215 ± 16.8 µm², n=47 nuclei) compared to untreated tumour spheres (488 ± 12.4 µm², n=230 nuclei) (Figure 3Q&S).

### Transcriptional profiling shows tumour spheres treated with a tRF-Gly-TCC mimic alter their gene expression when compared to primary tumours

In order to generate a molecular picture of clonal evolution, we performed a review of the literature to create a functional interaction map of the transcripts involved (Figure 4). We selected several candidate genes from the map to investigate gene expression across primary tumours to use as a reference to compare the treated tumour spheres in order to gain insight of the observed phenotypic effects at a molecular level. SRY-box 9 (SOX9) is a chondrocyte differentiation marker. We found SOX9 expression in control tissue but reduced expression in all tumour grades and remains at a reduced expression with the tRF-Gly-TCC mimic when compared to control (Figure 5A). Histone deacetylase 4 (*HDAC4*) is a negative regulator of gene expression via modification of chromatin structure. We found *HDAC4* has a lower expression in low and intermediate grade tumours when compared to controls (Figure 5B). *HDAC4* expression increases in high grade tumours and increases further in tumour spheres treated with a tRF-Gly-TCC mimic (Figure 5B). Runt related transcription factor 2 (*RUNX2*) is an embryonic regulator of primitive bone cell proliferation and migration. We found *RUNX2* steadily increases in expression throughout chondrosarcoma progression, reaching a forty-fold higher expression in high grade tumours when compared to controls (Figure 5C). *RUNX2* expression is reduced to the same level of expression as control tissue when tumour spheres are treated with the tRF-Gly-TCC mimic (Figure 5C). Parathyroid hormone-like protein (*PTHLP or PTHrP*) is a marker of immature chondrocytes and propagates the secondary ossification centre. We found *PTHLP* is expressed in control tissue, has a reduced expression in low grade tumours with a gradual increase in expression throughout chondrosarcoma progression (Figure 5D). Expression of *PTHLP* is lost in the tumour spheres treated with a tRF-Gly-TCC mimic (Figure 5D).

*ANG* is responsible for cellular hypoxic stress response, angiogenesis and production of some tRFs. We found *ANG* has a reduced expression in low and high tumour grades, in intermediate grade it is expressed similarly to control levels (Figure 5E). This pattern of ANG expression is consistent with tRF-Gly-TCC production. Indian hedgehog (*IHH*) is involved in chondrocyte differentiation, maturation and proliferation. We found *IHH* expression is similar to that of *ANG* where there is a reduction of expression across tumour grades except for intermediate grade tumours where expression exceeds that of control tissues (Figure 5F). In both *ANG* and *IHH,* expression is greatly reduced in tumour spheres treated with a tRF-Gly-TCC mimic (Figure 5E&F). *YBX1,* the RNA binding protein, increases in expression throughout chondrosarcoma progression (Figure 5G). Tumour spheres treated with tRF-Gly-TCC show increased expression of *YBX1* (Figure 5G). Tumour protein p53 (*TP53*) is the most well-known tumour suppressor protein where its master role is to regulate senescence and apoptosis. *TP53* gradually increases in expression throughout chondrosarcoma progression, peaking in intermediate grade tumours and reducing slightly in high grade tumours (Figure 5H). *TP53* expression reduces to be consistent with control tissues in tumour spheres treated with a tRF-Gly-TCC mimic (Figure 5H).

### Inhibition of RUNX2 binding to DNA in bone 3D tumour spheres using the small molecule (formula (I)) leads to a significant reduction of cancer cell number compared to controls

The results of treatment of bone 3D tumour spheres using 3-(N-(3,4-dichlorophenyl)carbamoyl)-5-norbornene-2-carboxylic acid, also named 3-{[(3,4-dichlorophenyl)amino]carbonyl}bicyclo[2.2.1]hept-5-ene-2-carboxylic acid, known as CADD522, a small molecule representative or prototypic of formula (I), at treatment concentrations of 50 µM and 100 µM small molecule are shown in Figure 8(b), against two controls. As was shown in Figure 5(c) (reproduced as Figure 8(a) for comparison), downregulation of *RUNX2* using an RNA inhibitor reduces *RUNX2* expression to control levels. Figure 8(b) shows that inhibition of RUNX2 binding to DNA in bone 3D tumour spheres using the small molecule of formula (I) leads to a significant reduction of cancer cell number compared to controls (two biological replicate controls). Statistical significance (**p* = <0.002 and ***p* = <0.005) was calculated using an unpaired t test with Prism 6 (GraphPad) software. The treatment at 50 µM CADD522 reduced the cancer cell number to about 70 % in comparison with Control 1 and the treatment at 100 µM CADD522 reduced the cancer cell number to about 30 % in comparison with Control 1. The 50% cell count mark in comparison with Control 1 is indicated on Figure 8(b).

### DISCUSSION

By integrating clinical, molecular, computational and phenotypic analysis, we describe a modulated set of tRNAs, tRFs and miRNAs associated with chondrosarcoma progression. We also show that the class of inhibitors of RUNX2 activity represented by formula (I) and salts, esters and prodrugs thereof (particularly, the compounds of formula (I) and pharmaceutically acceptable salts, esters and prodrugs thereof) inhibit RUNX2 binding to DNA in bone 3D tumour (chondrosarcoma) spheres leading to a significant reduction of cancer/tumour cell number compared to controls.

One of the identified miRNAs, miR-140, is highly expressed in high grade chondrosarcoma. Our previous work in embryonic bone development shows miR-140 is a key driver of primitive mesenchymal cell proliferation and migration through *HDAC4* and *RUNX2* (Green, D., Dalmay, T. & Fraser, W.D. Role of miR-140 in embryonic bone development and cancer. Clin Sci (Lond) 129, 863-73 (2015), Tuddenham, L. et al. The cartilage specific microRNA-140 targets histone deacetylase 4 in mouse cells. FEBS Lett 580, 4214-7 (2006), Nicolas, F.E. et al. Experimental identification of microRNA-140 targets by silencing and overexpressing miR-140. RNA 14, 2513-20 (2008), Nicolas, F.E. et al. mRNA expression profiling reveals conserved and non-conserved miR-140 targets. RNA Biol 8, 607-15 (2011), Pais, H. et al. Analyzing mRNA expression identifies Smad3 as a microRNA-140 target regulated only at protein level. RNA 16, 489-94 (2010), Nicolas, F.E. & Dalmay, T. New evidence supports a role of MiR-140 at early stages of bone development. Arthritis Rheum (2013)). MiR-140 negatively regulates *HDAC4,* which leads to the increase in expression of *RUNX2* (Green, D., Dalmay, T. & Fraser, W.D. Role of miR-140 in embryonic bone development and cancer. Clin Sci (Lond) 129, 863-73 (2015), Tuddenham, L. et al. The cartilage specific microRNA-140 targets histone deacetylase 4 in mouse cells. FEBS Lett 580, 4214-7 (2006), Nicolas, F.E. & Dalmay, T. New evidence supports a role of MiR-140 at early stages of bone development. Arthritis Rheum (2013)). In the present study, we find *RUNX2* is expressed forty-fold higher in high grade tumours when compared to controls. It has been shown miR-320a targets *RUNX2* for silencing, which inhibits immature chondrocyte proliferation and migration (Hamam, D. et al. microRNA-320/RUNX2 axis regulates adipocytic differentiation of human mesenchymal (skeletal) stem cells. Cell Death Dis 5, e1499 (2014)). MiR-486 has previously been reported to drive proliferation and reduce apoptosis in response to hypoxia by upregulating the expression of *VEGF* in mesenchymal stem cells (Shi, X.F. et al. MiRNA-486 regulates angiogenic activity and survival of mesenchymal stem cells under hypoxia through modulating Akt signal. Biochem Biophys Res Commun 470, 670-7 (2016)). Taken together we show restoration of embryonic signalling in high grade chondrosarcoma.

We show that tRF-Gly-TCC is a tumour suppressor in chondrosarcoma. TRF-Gly-TCC expression is reduced in high grade tumours (Figure 2B). In line with previous data metastatic clones attenuate expression of specific tRFs (Goodarzi, H. et al. Endogenous tRNA-Derived Fragments Suppress Breast Cancer Progression via YBX1 Displacement. Cell 161, 790-802 (2015)). Sequence analysis of tRFs that are induced by hypoxia reveals significant enrichment of a common linear sequence motif SCUBYC (Goodarzi, H. et al. Endogenous tRNA-Derived Fragments Suppress Breast Cancer Progression via YBX1 Displacement. Cell 161, 790-802 (2015)). TRFs containing this motif, including tRF-Gly-TCC, are reduced in metastatic cells. TRFs that do not harbour this motif continue to be expressed. These seemingly deliberate modifications of the regulatory transcriptomic landscape demonstrate clonal evolution and selection for clones with metastatic propensity. A previous report investigating the SCUBYC sequence motif showed the motif to serve as a binding site for a common trans factor that interacts with tRFs in vivo. The RNA binding protein YBX1 consistently co-precipitated with the SCUBYC motif (Goodarzi, H. et al. Endogenous tRNA-Derived Fragments Suppress Breast Cancer Progression via YBX1 Displacement. Cell 161, 790-802 (2015)). TRFs interact with YBX1 to displace eukaryotic initiation factors 4B, 4E and 4G from the m⁷G cap of mRNA, which interferes with secondary structure confirmation during ribosome scanning (Green, D., Fraser, W.D. & Dalmay, T. Transfer RNA-derived small RNAs in the cancer transcriptome. Pflugers Arch (2016), Ivanov, P., Emara, M.M., Villen, J., Gygi, S.P. & Anderson, P. Angiogenin-induced tRNA fragments inhibit translation initiation. Mol Cell 43, 613-23 (2011), Harms, U., Andreou, A.Z., Gubaev, A. & Klostermeier, D. eIF4B, eIF4G and RNA regulate elF4A activity in translation initiation by modulating the elF4A conformational cycle. Nucleic Acids Res 42, 7911-22 (2014)). TRFs further competitively interact with YBX1 leading to destabilisation of oncogenic mRNAs (Goodarzi, H. et al. Modulated Expression of Specific tRNAs Drives Gene Expression and Cancer Progression. Cell 165, 1416-27 (2016), Goodarzi, H. et al. Endogenous tRNA-Derived Fragments Suppress Breast Cancer Progression via YBX1 Displacement. Cell 161, 790-802 (2015), Green, D., Fraser, W.D. & Dalmay, T. Transfer RNA-derived small RNAs in the cancer transcriptome. Pflugers Arch (2016)). TRF-Gly-TCC specifically displayed a substantial binding to YBX1 (Goodarzi, H. et al. Endogenous tRNA-Derived Fragments Suppress Breast Cancer Progression via YBX1 Displacement. Cell 161, 790-802 (2015)). By attenuating the induction of tRFs containing a SCUBYC sequence motif, metastatic cells evade tRF-YBX1 interaction and subsequent tumour suppression.

We find that remodulating the cancer transcriptome with tRF-Gly-TCC induces phenotypic and transcription changes in a model system. We speculate the transfected sequences overcome YBX1, terminating the stability of oncogenic messenger RNAs and inhibiting the role of YBX1 in cancer progression (Figure 7). YBX1 is one of the most overexpressed oncogenes observed in human cancer (upregulated in 10% of all cancer versus normal tissue data sets) and is highly heterogeneous with multiple variants/post-translational modifications (Rhodes, D.R. et al. ONCOMINE: a cancer microarray database and integrated data-mining platform. Neoplasia 6, 1-6 (2004), Uchiumi, T. et al. YB-1 is important for an early stage embryonic development: neural tube formation and cell proliferation. J Biol Chem 281, 40440-9 (2006)). A number of YBX1 target transcripts encode well characterised drivers of oncogenesis including eukaryotic translation initiation factor 4 gamma 1 (EIF4G1), integrin subunit beta 4 (ITGB4), AKT serine/threonine kinase 1 (AKT1) and ADAM metallopeptidase domain 8 (ADAM8) (Goodarzi, H. et al. Endogenous tRNA-Derived Fragments Suppress Breast Cancer Progression via YBX1 Displacement. Cell 161, 790-802 (2015)). Sequence analysis of *RUNX2,* the embryonic transcript that steadily increases in expression inversely to tRF-Gly-TCC, reveals several YBX1 recognition motifs in the coding and 3' regions (Figure 6). Transfecting tRF-Gly-TCC reduces *RUNX2* expression to control levels. Our study shows the potential of modulating the interaction of YBX1 through molecular manipulation rather than inducing massive cell death, which is the objective of cytotoxic chemotherapy.

Few transcriptomic analyses have been performed in chondrosarcoma that may contain insights into endogenous regulatory mechanisms that could be therapeutically manipulated. In this study we show miRNAs are differentially expressed throughout chondrosarcoma progression. One miRNA, miR-140, is highly expressed in high grade chondrosarcoma and known to interact with *HDAC4* and downstream with *RUNX2* during embryonic bone development. We also show a recently discovered class of sRNA known as tRFs are differentially expressed throughout chondrosarcoma progression. Ectopic expression of tRF-Gly-TCC in chondrosarcoma tumour spheres induced cellular and genetic changes that are visibly and experimentally different to controls. Our data expands the importance of RNA silencing in cancer biology and demonstrates a novel role for tRNAs and tRFs in actively regulating gene expression. Manipulating this endogenous transcriptomic defence mechanism is a novel approach to treating bone cancer.

We have also shown that the class of inhibitors of RUNX2 activity represented by formula (I) and salts, esters and prodrugs thereof (particularly, the compounds of formula (I) and pharmaceutically acceptable salts, esters and prodrugs thereof) inhibit RUNX2 binding to DNA in bone 3D tumour (chondrosarcoma) spheres leading to a significant reduction of cancer/tumour cell number compared to controls.

Taking the above data together, the compositions and uses of the present invention and the methods of the disclosure are demonstrated to be effective against cartilage matrix-forming bone tumours and/or metastatic cancer originating therefrom, for example chondrosarcoma and metastatic chondrosarcoma.

The foregoing broadly describes certain embodiments of the present invention without limitation. Variations and modifications as will be readily apparent to those skilled in the art are intended to be within the scope of the present invention as defined in and by the appended claims.

## Claims

1. A composition for use in a method of treating a chondrosarcoma and/or a metastatic cancer originating from a chondrosarcoma, wherein the composition comprises an inhibitor of RUNX2 activity, wherein the inhibitor of RUNX2 activity is a compound according to formula (I) or a pharmaceutically acceptable salt, ester or prodrug thereof, 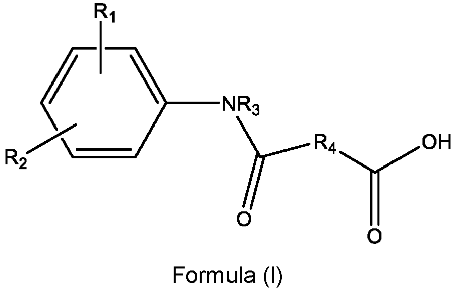
wherein R₁ and R₂ are each independently selected from hydrogen, a halogen, a haloalkyl, an alkyl, an alkylamide, a cycloalkylamide or an alkyamine;
R₃ is H or alkyl; and
R₄ is a bridged cycloalkenyl ring.

2. The composition for the use of claim 1, wherein the chondrosarcoma is a cartilage matrix-forming bone cancer.

3. The composition for the use of claim 1 or 2, wherein metastasis of the chondrosarcoma is inhibited.

4. The composition for the use of any preceding claim, wherein the chondrosarcoma overexpresses RUNX2 in comparison to normal cartilage or other biological material.

5. The composition for the use of any preceding claim, wherein the compound of formula (I) is 3-(N-(3,4-dichlorophenyl)carbamoyl)-5-norbornene-2-carboxylic acid, also named 3-{[(3,4-dichlorophenyl)amino]carbonyl}bicyclo[2.2.1]hept-5-ene-2-carboxylic acid, known as CADD522 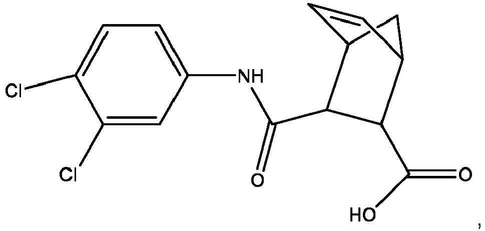 or a salt, ester or prodrug thereof.

## Patentansprüche

1. Zusammensetzung zur Verwendung in einem Verfahren zur Behandlung eines Chondrosarkoms und/oder eines von einem Chondrosarkom stammenden metastatischen Krebses, wobei die Zusammensetzung einen Inhibitor der RUNX2-Aktivität umfasst, wobei der Inhibitor der RUNX2-Aktivität eine Verbindung gemäß Formel (I) oder ein pharmazeutisch verträgliches Salz, Ester oder Pro-Pharmakon davon ist, 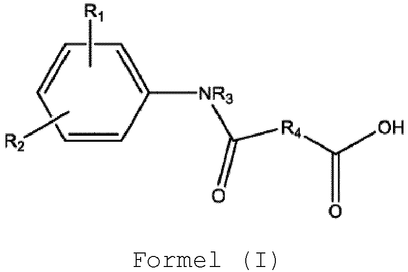
wobei R₁ und R₂ unabhängig voneinander ausgewählt sind aus Wasserstoff, einem Halogen, einem Halogenalkyl, einem Alkyl, einem Alkylamid, einem Cycloalkylamid oder einem Alkylamin;
R₃ H oder Alkyl ist; und
R₄ ein überbrückter Cycloalkenylring ist.

2. Zusammensetzung zur Verwendung nach Anspruch 1, wobei das Chondrosarkom ein Knorpelmatrix-bildender Knochenkrebs ist.

3. Zusammensetzung zur Verwendung nach Anspruch 1 oder 2, wobei die Metastasierung des Chondrosarkoms gehemmt wird.

4. Zusammensetzung zur Verwendung nach einem der vorstehenden Ansprüche, wobei das Chondrosarkom RUNX2 im Vergleich zu normalem Knorpel oder anderem biologischem Material überexprimiert.

5. Zusammensetzung zur Verwendung nach einem der vorstehenden Ansprüche, wobei die Verbindung der Formel (I) 3-(N-(3,4-Dichlorphenyl)carbamoyl)-5-norbornen-2-carbonsäure, auch 3-{[(3,4-Dichlorphenyl)amino]carbonyl}bicyclo[2.2.1]hept-5-en-2-carbonsäure genannt, bekannt als CADD522, 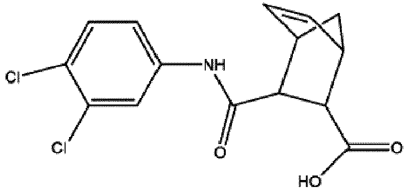 oder ein Salz, Ester oder Pro-Pharmakon davon ist.

## Revendications

1. Composition destinée à être utilisée dans une méthode de traitement d'un chondrosarcome et/ou d'un cancer métastatique provenant d'un chondrosarcome, dans laquelle la composition comprend un inhibiteur de l'activité RUNX2, dans laquelle l'inhibiteur de l'activité RUNX2 est un composé selon la formule (I) ou un sel, ester ou promédicament pharmaceutiquement acceptables de celui-ci, 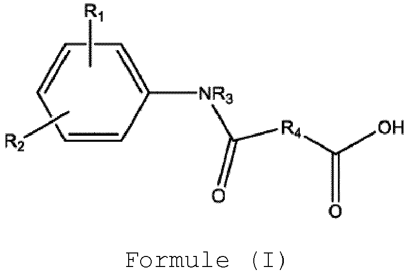
dans laquelle R₁ et R₂ sont chacun indépendamment choisis parmi l'hydrogène, un halogène, un halogénoalkyle, un alkyle, un alkylamide, un cycloalkylamide ou une alkyamine ;
R₃ est H ou alkyle ; et
R₄ est un cycle cycloalcényle ponté.

2. Composition destinée à être utilisée selon la revendication 1, dans laquelle le chondrosarcome est un cancer osseux formant une matrice cartilagineuse.

3. Composition destinée à être utilisée selon la revendication 1 ou 2, dans laquelle la métastase du chondrosarcome est inhibée.

4. Composition destinée à être utilisée selon une quelconque revendication précédente, dans laquelle le chondrosarcome surexprime RUNX2 par rapport au cartilage normal ou à un autre matériel biologique.

5. Composition destinée à être utilisée selon une quelconque revendication précédente, dans laquelle le composé de formule (I) est l'acide 3-(N-(3,4-dichlorophényl)carbamoyl)-5-norbornène-2-carboxylique, également appelé acide 3-{[(3,4-dichlorophényl)amino]carbonyl}bicyclo[2.2.1]hept-5-ène-2-carboxylique, connu sous le nom de CADD522 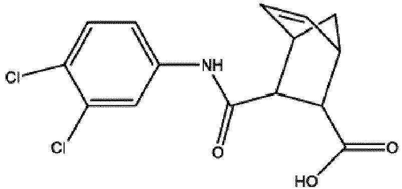 ou un sel, ester ou promédicament de celui-ci.
